(19) Europäisches Patentamt
European Patent Office
Office européen des brevets

(11) Numéro de publication: **0 171 315**
**B1**

(12) **FASCICULE DE BREVET EUROPEEN**

(45) Date de publication du fascicule du brevet: **18.01.89**

(51) Int. Cl.[4]: **C 07 K 7/10**, C 07 K 5/00, A 61 K 37/02

(21) Numéro de dépôt: **85401376.0**

(22) Date de dépôt: **08.07.85**

(54) **Procédé de synthèse du hGRF (Somatocrinine) en phase liquide et peptides intermédiaires.**

(30) Priorité: **10.07.84 FR 8410965**

(43) Date de publication de la demande:
**12.02.86 Bulletin 86/7**

(45) Mention de la délivrance du brevet:
**18.01.89 Bulletin 89/3**

(84) Etats contractants désignés:
**AT BE CH DE FR GB IT LI LU NL SE**

(73) Titulaire: **SANOFI, société anonyme, 40, Avenue George V, F-75008 Paris (FR)**

(72) Inventeur: **Diaz, Joseph, 19 rue du Pradas, F-34470 Perols (FR)**
Inventeur: **Demarne, Henri, Le Florence 65 Avenue Major Flandre, F-34000 Montpellier (FR)**
Inventeur: **Roncucci, Roméo, 20 rue Tournefort, F-75005 Paris (FR)**
Inventeur: **Schmelck, Paul-Henry, 16 rue Viala, F-31520 Ramonville Saint-Agne (FR)**

(74) Mandataire: **Gillard, Marie-Louise et al, Cabinet Beau de Loménie 55, Rue d'Amsterdam, F-75008 Paris (FR)**

(56) Documents cités:
**EP-A- 0 105 759**
**EP-A- 0 107 890**
**EP-A- 0 117 034**
**EP-A- 0 122 818**

**CHEMICAL ABSTRACTS, vol. 101, 1984, page 80, no. 104311x, Columbus, Ohio, US; N. LING et al.: "Isolation, primary structure, and synthesis of human hypothalamic somatocrinin: growth hormone-releasing factor" & PROC. NATL. CAD. SCI. U.S.A. 1984, 81(14), 4302-6**
**CHEMICAL ABSTRACTS, vol. 101, 1984, page 86, no.**

(56) Documents cités: (suite)
**144228g, Columbus, Ohio, US; R. GUILLEMIN et al.: "Somatocrinin, the growth hormone releasing factor" & RECENT PROG. HORM. RES. 1984, 40, 233-99**
**CHEMICAL & PHARMACEUTICAL BULLETIN, vol. 32, no. 2, 1984, pages 520-529, Pharmaceutical Society of Japan; N. FUJII et al.: "Studies on peptides. Solution synthesis of the tetratetracontapeptide amide corresponding to the entire amino acid sequence of growth hormone releasing factor, somatocrinin"**

**EP 0 171 315 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Le hGRF (Human Growth Hormone releasing factor) ou Somatocrinine est un peptide constitué par l'enchaînement de 44 amino acides. Sa séquence est la suivante:

```
1                    5                        10
H – Tyr – Ala – Asp – Ala – Ile – Phe – Thr – Asn – Ser – Tyr – Arg
              15                   20
Lys – Val – Leu – Gly – Gln – Leu – Ser – Ala – Arg – Lys – Leu – Leu
  25                        30                       35
Gln – Asp – Ile – Met – Ser – Arg – Gln – Gln – Gly – Glu – Ser – Asn
                 40                   44
Gln – Glu – Arg – Gly – Ala – Arg – Ala – Arg – Leu – NH2
```

Il a été récemment découvert par A. Guillemin et coll. (Science 218, 585–587 (1982) à partir d'extraits d'une tumeur pancréatique humaine.

N. Ling et al. (Proc. Natl. Acad. Sci. USA 1984, 81 (14), 4302–6) ont décrit l'extraction et la purification du GRF à partir de tissus hypophysaires ainsi que la détermination de la séquence des amino-acides constituant ce peptide.

La structure et l'activité biologique du GRF a été décrite par R. Guillemin et al. (Recent. Prog. Horm. Rests. 1984, 40 233–99). Les auteurs signalent dans cet article que le GRF et certains de ses fragments ont été préparés par synthèse en phase solide.

Ce peptide est particulièrement actif sur la stimulation de la libération de l'hormone de croissance (GH) aussi bien in vitro qu'in vivo. In vitro, en particulier son efficacité se manifeste à des doses de quelques femtomoles/ml. ($ED_{50}$ = 15 femtomoles/ml). L'intérêt thérapeutique en médecine humaine de cette substance va donc se situer au niveau du traitement du nanisme et des retards à la croissance en pédiatrie. D'autres applications sont possibles dans les cas de déficience anabolique protéique (ulcères de stress, réparation de fractures ou d'atteintes du cartilage, brulûres étendues (pendant la phase anabolique), réparations cutanées, ostéoporoses).

Dans le domaine vétérinaires, ce composé présente un intérêt évident au niveau de la croissance pondérale des animaux d'élevage (bovins, ovins, porcins, poulets, etc.) et de l'augmentation de la lactation (bovins, ovins, etc.)

Le développement industriel de ce composé polypeptidique nécessite la synthèse de quantités importantes de cette substance. Les procédés classiques de la synthèse en phase solide permettent la préparation en des temps courts de faibles quantités de ce principe actif (Science, 218, 585–587 (1982) à des coûts très élevés et incompatibles avec un développement pharmaceutique à grande échelle.

Le brevet EP-A 107 890 concerne le GRF et certains de ses analogues. Bien que d'une façon générale il soit indiqué que ces peptides peuvent être préparés par l'une des méthodes connues en synthèse peptidique, seule la méthode en phase solide est illustrée dans les exemples.

Le brevet EP-A 105 759 concerne le GRF (1–40) et un procédé pour son obtention, qui consiste en un enchaînement classique d'amino-acides sans faire appel à des fragments particuliers.

Le brevet EP-A 117 034 concerne des fragments et analogues du GRF à l'exclusion du GRF lui-même. Au point de vue de la synthèse des peptides, seule la méthode en phase solide est illustrée.

L'article FUJI et al. dans Chemical et pharmaceutical Bulletin 32 (2), Février 1984 pages 520–529 concerne une synthèse en solution du GRF mettant en oeuvre huit fragments peptidiques dont le fragment 23–27, enchaînés séquentiellement de façon à former la chaîne polypeptidique souhaitée, la technique de condensation utilisée étant celle aux hydrazides.

Dans son brevet européen no. 122818 la demanderesse a déjà décrit un procédé de synthèse en phase liquide de la somatocrinine permettant d'obtenir des quantités importantes de ce produit.

La présente demande a pour objet un nouveau procédé de synthèse permettant d'améliorer considérablement la pureté des peptides intermédiaires formés et par voie de conséquence de faciliter les phases de purification finale et d'améliorer nettement les rendements en somatocrinine pure.

Le procédé suivant la présente invention fait intervenir les fragments suivants:

| | | | |
|---|---|---|---|
| H–Ala–Arg–Ala–Arg–Leu–$NH_2$ | fragment A | hGRF | (40–44) |
| H–Gln–Glu–Arg–Gly–OH | fragment $B'_1$ | hHGRF | (36–39) |
| H–Glu–Ser–Asn–OH | fragment $B'_2$ | hGRF | (33–35) |
| H–Ser–Arg–Gln–Gln–Gly–OH | fragment C | hGRF | (28–32) |
| H–Leu–Gln–Asp–Ile–Met–OH | fragment D' | hGRF | (23–27) |
| H–Arg–Lys–Leu–OH | fragment $E'_1$ | hGRF | (20–22) |
| H–Gln–Leu–Ser–Ala | fragment $F_1$ | hGRF | (16–19) |

| | | | |
|---|---|---|---|
| H–Tyr–Arg–Lys–Val–Leu–Gly–OH | fragment $G_1$ | hGRF | (10–15) |
| H–Ile–Phe–Thr–Asn–Ser–OH | fragment $H_1$ | hGRF | ( 5–9) |
| H–Tyr–Ala–Asp–Ala–OH | fragment I | hGRF | ( 1–4) |

On couple ensuite les fragments A, $B'_1$, $B'_2$, C, D', $E'_1$ dans l'ordre de la séquence de hGRF pour obtenir le peptide $K_1$ (hGRF (20–44) et les fragments $F_1$, $G_1$, $H_1$ également dans l'ordre de la séquence du GRF pour obtenir le peptide J hGRF (5–19).

Finalement on couple dans l'ordre:

| | |
|---|---|
| 5–19 + 20–44 | 5–44 |
| 1–4 + 5–44 | 1–44 |

Le procédé de la présente invention est caractérisé, en outre, en ce que l'on couple, l'un après l'autre et dans l'ordre de la séquence, les fragments dans lesquels:

a) les fonctions acides latérales des acides aspartique et glutamique et la fonction amine latérale de la lysine sont protégées par des groupes protecteurs stables dans les conditions de déprotection du groupement Boc (tertiobutyloxycarbonyle)

b) la fonction guanidine de l'arginine est protégée par protonation, et

c) l'acide aminé N-terminal est protégé sur l'amine par le groupement Boc

d) en éliminant sélectivement le groupement Boc de l'amine N-terminale du peptide en phase d'élongation par hydrolyse à l'acide trifluoracétique, ledit couplage étant effectué dans un solvant polaire aprotique et on élimine en fin de séquence tous les groupes protecteurs par hydrolyse à l'aide d'une solution 0,1 à 1 M d'acide méthanesulfonique ou trifluorométhanesulfonique dans l'acide trifluoroacétique.

Le procédé de synthèse du GRF se caractérise en outre par les aspects suivants:

– Application du principe de la protection minimale au niveau des chaînes latérales fonctionnalisées.

– A l'exception de l'arginine en position 20, on effectue une protection temporaire de la fonction guanidine latérale de l'arginine par le groupement nitro. L'arginine est introduite en séquence sous forme nitro guanidine. La fonction nitro est ensuite éliminée le plus tôt possible (voir synthèse des fragments) par hydrogénation catalytique à l'aide de Pd/charbon, ou bien en utilisant un générateur d'hydrogène comme l'acide formique ou le formiate d'ammonium. Ainsi, excepté la position 20, tous les fragments synthétisés possédant de l'arginine dans leur séquence ont en fin de synthèse les fonctions guanidine, simplement protégées par protonation à l'aide d'un acide fort (acide chlorhydrique par exemple).

– La fonction guanidine de l'arginine en position 20 est protégée par le groupement MTS (triméthyl–2,4,6 phénylsulfonyle ou mésitylsulfonyle).

– Les fonctions acides carboxyliques des chaînes latérales des acides glutamique et aspartique sont protégées par des groupements clivables par hydrogénation catalytique ($H_2$/Pd/charbon) ou bien en milieu acide fort comme les mélanges acide méthanesulfonique (0,5 M) – acide trifluoroacétique, ou acide trifluorométhanesulfonique (0,5 M) – acide trifluoroacétique. Nous préconisons comme protecteurs l'ester benzylique (0 Bzl) ou 2,6-dichlorobenzylique. Ces groupements protecteurs sont stables dans les conditions des déprotections intermittentes des amines en α (élimination des groupements t-butyloxycarbonyle: Boc par l'acide trifluoroacétique)– les fonctions amines des chaînes latérales des lysines sont protégées par des groupements clivables dans les mêmes conditions que précédemment. Nous préconisons les groupements benzyloxycarbonyle (Z), 2-chloro ou 2-bromo-benzyloxycarbonyle (2 – Cl ou 2 – Br Z) stables dans les conditions de déprotection intermittente par l'acide trifluoroacétique.

Les fonctions hydroxyle présentes dans la thréonine, la sérine et la tyrosine ne sont pas protégées.

L'élongation du peptide à partir des fragments synthétisés est réalisée en utilisant comme agent de couplage l'hexafluorophosphate de benzotriazolyl-oxyphosphonium (BOP), ou le dicyclohexylcarbodiimide en présence d'hydroxy-1 benzotriazole, ou selon la méthode aux carboxyazides (Curtius) dans un solvant approprié comme le diméthylformamide ou de diméthylsulfoxyde. L'isolement du produit du milieu réactionnel est effectué par introduction d'un tiers solvant insolubilisant (éther, acétate d'éthyle ...) qui précipite le peptide.

On se limite pendant les étapes de couplage à des purifications sommaires du type lavages en phase solide à l'aide de solvants appropriés afin d'éliminer le léger excès du dernier fragment couplé, ainsi que les impuretés apportées par les agents de couplage.

Chaque opération de couplage d'un fragment est suivie d'une phase de déprotection intermittente du groupement protecteur Boc (tertio-butyloxycarbonyle) au niveau de l'amine sur laquelle va être effectué le couplage suivant. Cette déprotection est assurée par l'acide trifluoroacétique dans le chlorure de méthylène (50/50 en volume).

Les déprotections des chaînes latérales en fin d'élongation du peptide peuvent être effectuées par hydrogénation en présence d'un catalyseur (comme le Pd/C) à l'aide d'hydrogène gazeux sous légère pression (1 à 5 kg) ou bien avec un générateur d'hydrogène comme l'acide formique ou le formiate d'ammonium. Il est également possible d'éliminer ce type de groupements protecteurs par un acide fort comme les mélanges d'acide méthanesulfonique dans l'acide trifluoroacétique (0,5 M) ou bien d'acide trifluorométhanesulfonique dans l'acide trifluoroacétique(0,5 M).

Le produit, après les déprotections terminales,

est purifié par filtration sur gel de Sephadex G 50 à l'aide de l'acide acétique à 30%. Les fractions enrichies en GRF 1–44 sont réunies et soumises à une chromatographie sur échangeurs d'ions (cations) du type carboxy et à l'aide d'un gradient à force ionique croissante adapté au type de résine échangeuse d'ions utilisée. Les fractions de plus haute pureté sont réunis et purifiées soit par chromatographie de partition sur un support approprié du type Sephadex G 50 ou Biogel P 10, soit par la distribution à contrecourant.

Les fractions dont le titre de pureté est jugé satisfaisant par HPLC analytique sont réunies. Les autres sont recyclées.

Une variante du procédé consiste à remplacer la chromatographie de partition ou la distribution à contre-courant par la HPLC préparative.

Le procédé de la présente invention peut aussi s'appliquer à la synthèse du GRF 1–40, produit naturel isolé également par R. Guillemin, presque aussi réactif que le GRF 1–44 et utilisable dans les mêmes indications thérapeutiques, auquel cas le fragment A [hGRF (40–44)] est remplacé dans la synthèse ci-dessus par l'alaninamide (H–Ala–$NH_2$).

L'invention concerne également les peptides ou fragments intermédiaires répondant aux formules ci-après:

- Boc – Gln – Glu–(O Bzl) – Arg – Gly – OH;
- X – Gln – Glu – Arg – Gly – OH;
- Boc – Glu – (O Bzl) – Ser – Asn – OH;
- X – Glu – Ser – Asn – OH;
- Boc – Arg(MTS) – Lys (Z) – Leu – O $CH_3$;
- X – Arg (MTS) – Lys (Z) – Leu – NH – $NH_2$;
- X – Gln – Glu (O Bzl) – Arg – Gly – Ala – Arg – Ala – Arg – Leu – $NH_2$;
- X – Glu (O Bzl) – Ser – Asn – Gln – Glu (O Bzl) – Arg – Gly – Ala – Arg – Ala – Arg – Leu – $NH_2$;
- X – Ser – Arg – Gln – Gln – Gly – Glu (O Bzl) – Ser – Asn – Gln – Glu (O Bzl) – Arg – Gly – Ala – Arg – Ala – Arg – Leu – $NH_2$;
- X – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$;
- X – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$;
- X – Ile – Phe – Thr – Asn – Ser – Tyr – Arg – Lys (Z) – Val – Leu – Gly – Gln – Leu – Ser – Ala – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$, dans lesquelles X représente H ou Boc.

L'invention a également pour objet le hGRF 1–44 protégé répondant à la formule ci-après:

- Y – Tyr – Ala – Asp (O Bzl) – Ala – Ile – Phe – Thr – Asn – Ser – Tyr – Arg – Lys (Z) – Val – Leu – Gly – Gln – Leu – Ser – Ala – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$, dans laquelle Y est H, Z ou Boc, ainsi que le hGRF 1–40 également protégé.

La stratégie de synthèse qui caractérise la présente invention se trouve résumée dans les schémas I, II et III, ci-après.

Les tableaux I à X donnent les schémas de synthèse des fragments de base:

Tableau I – (fragment A)

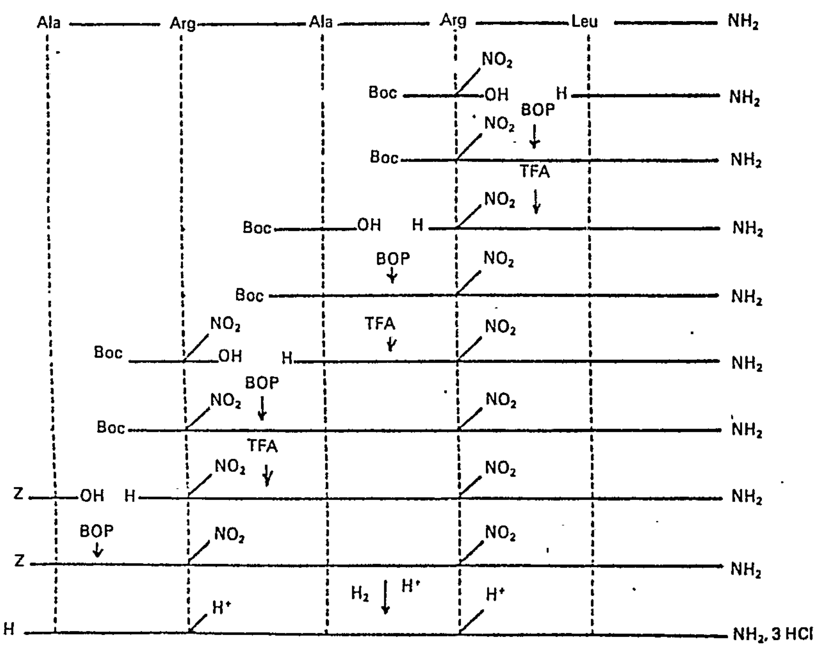

Tableau II (Fragment B'$_1$)

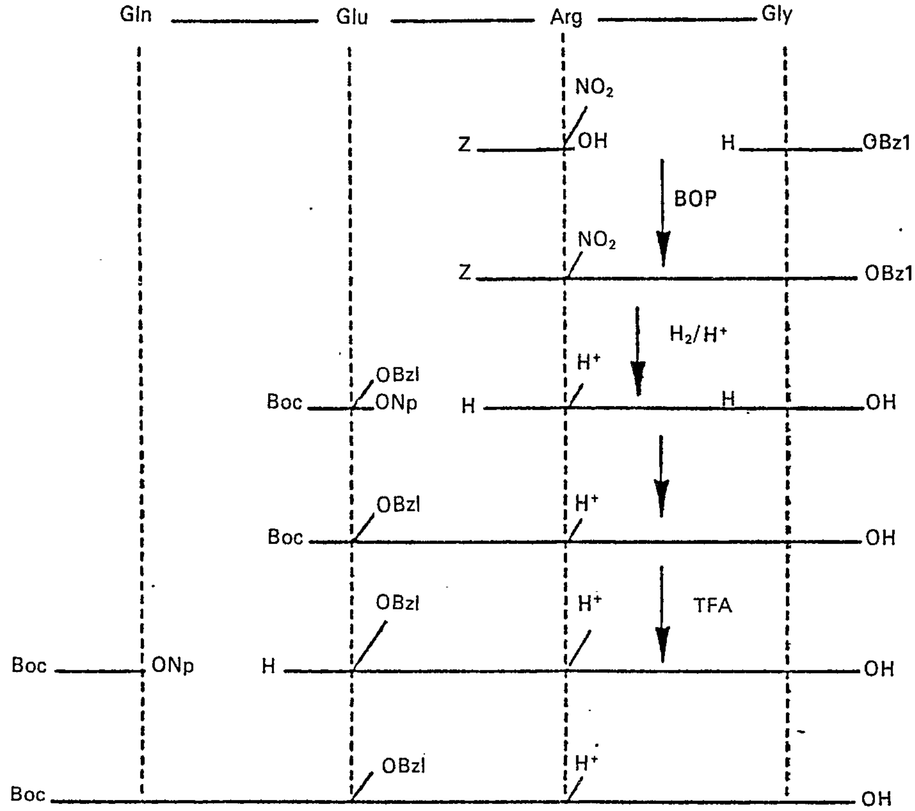


Tabbleau III (fragment B'$_2$)

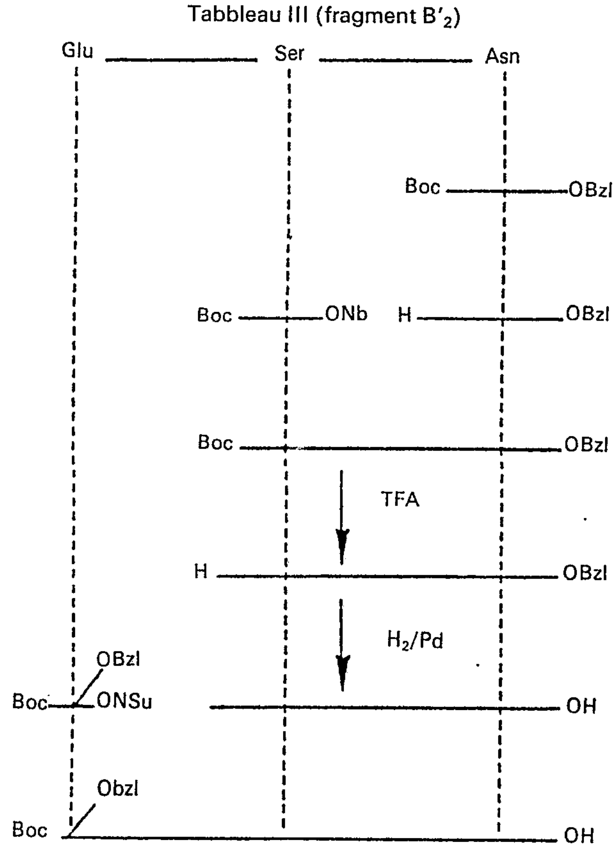

Tableau IV – (fragment C)

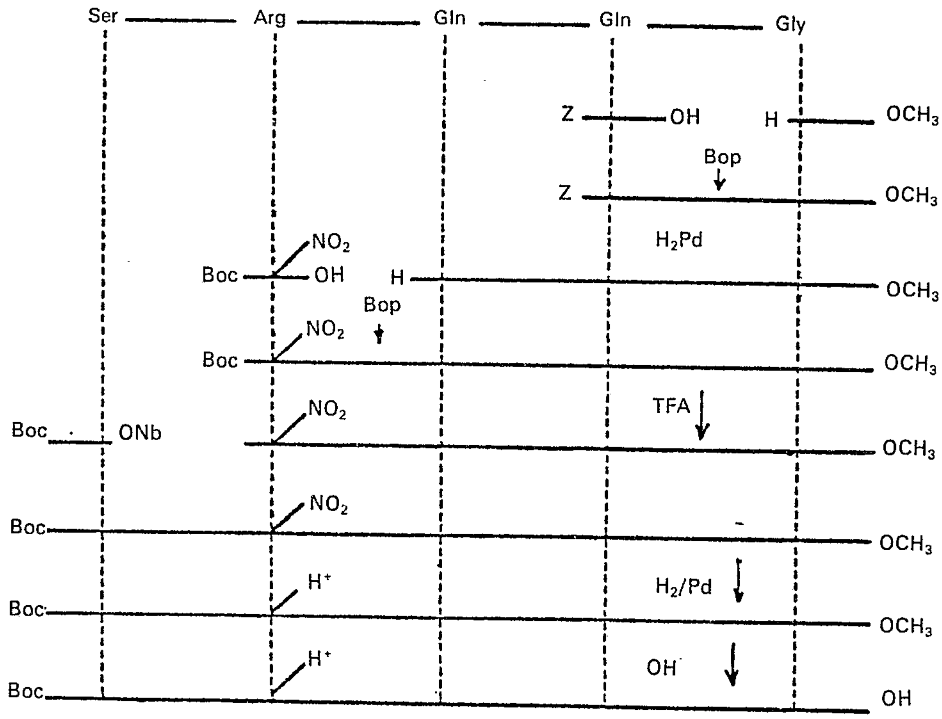


Tableau V – (fragment D')

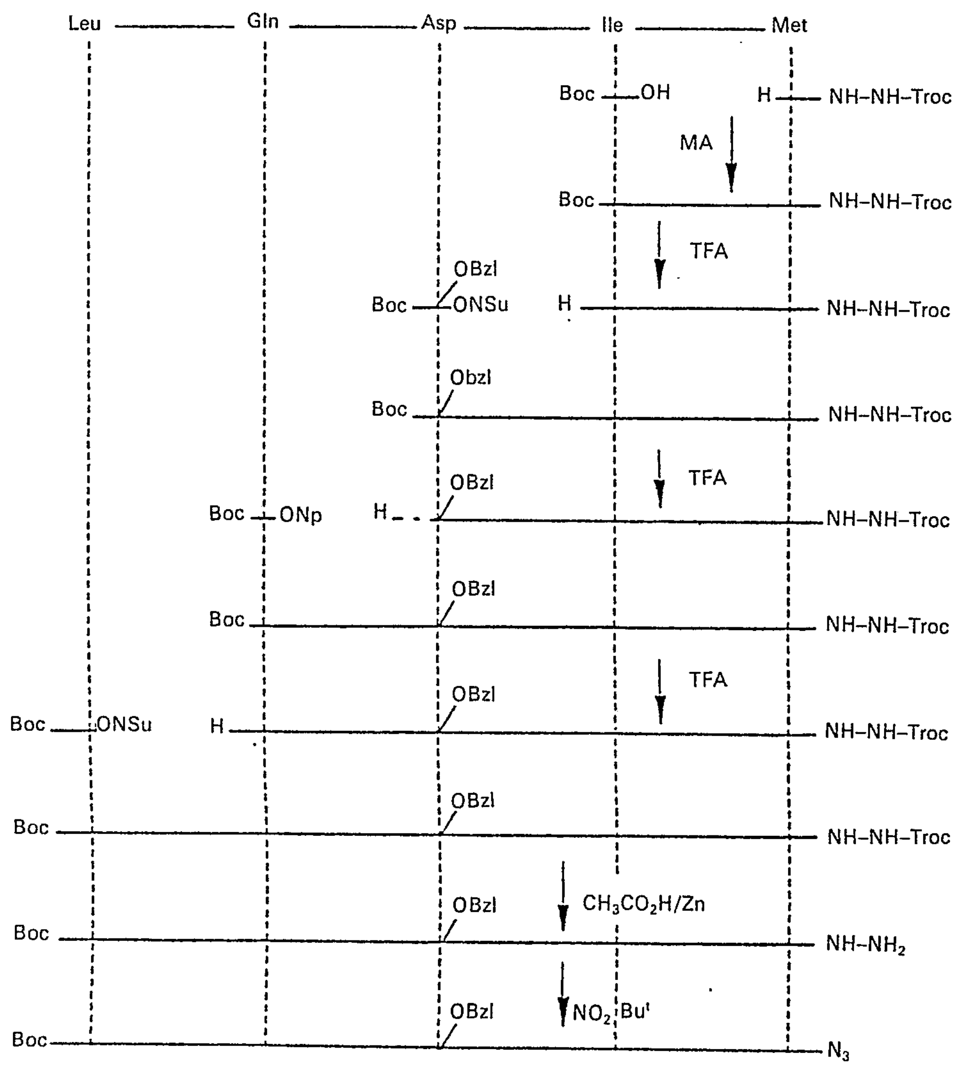

Tableau VI – (fragment E'$_1$)

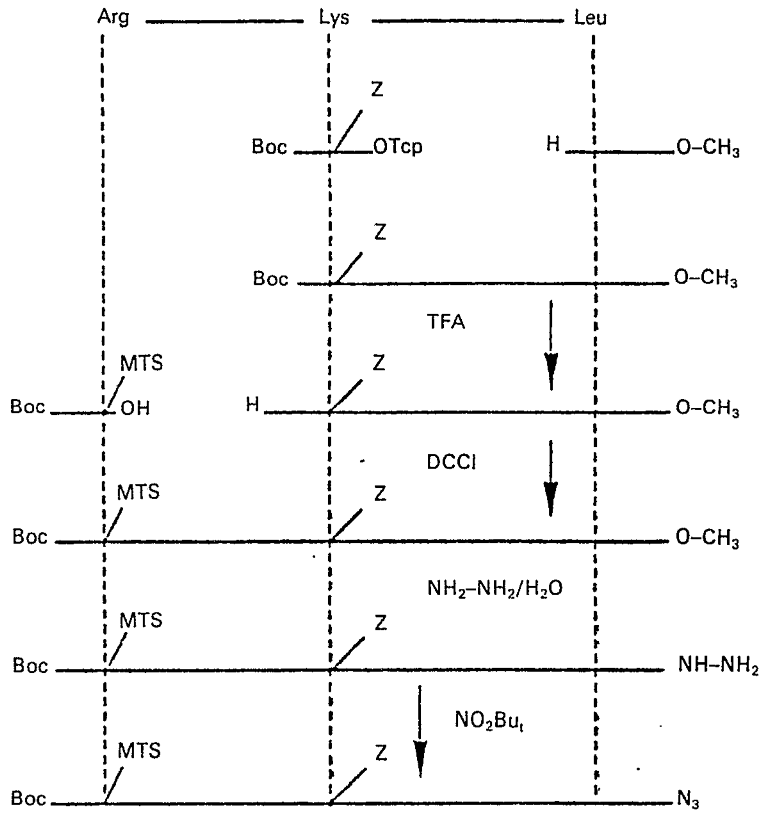


Tableau VII (fragment F$_1$)

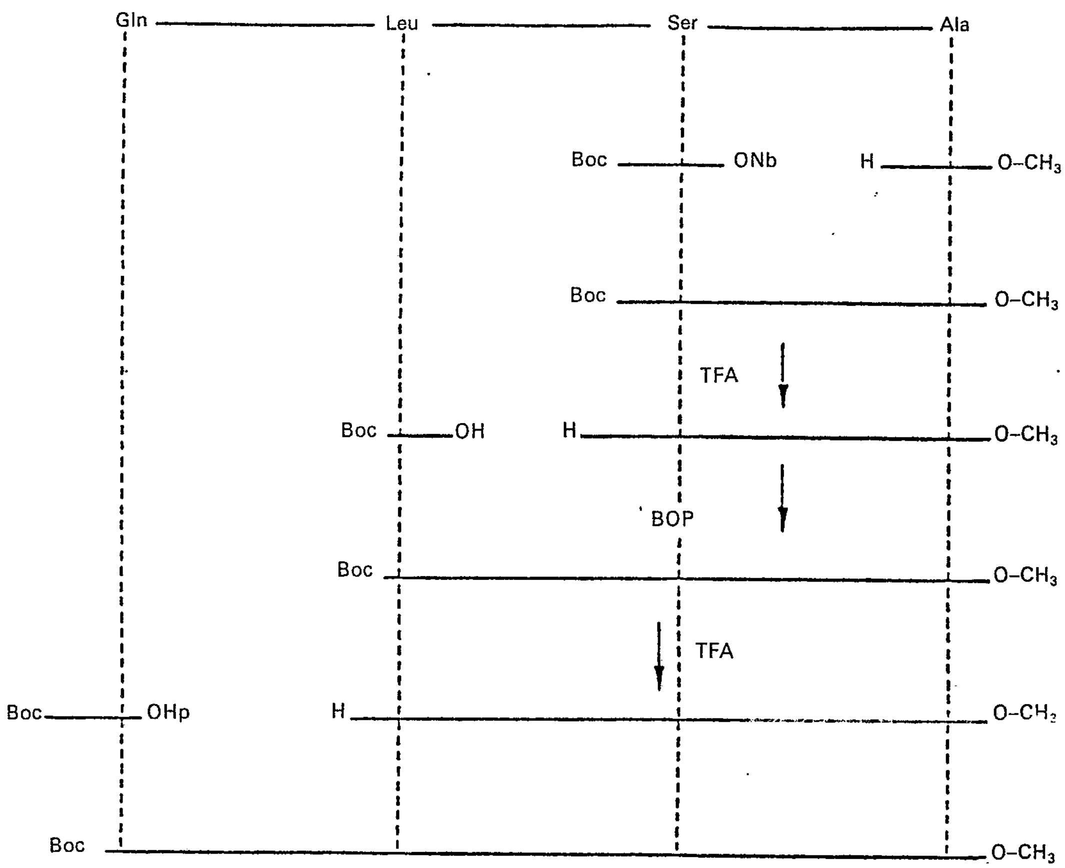

Tableau VIII (fragment $G_1$)

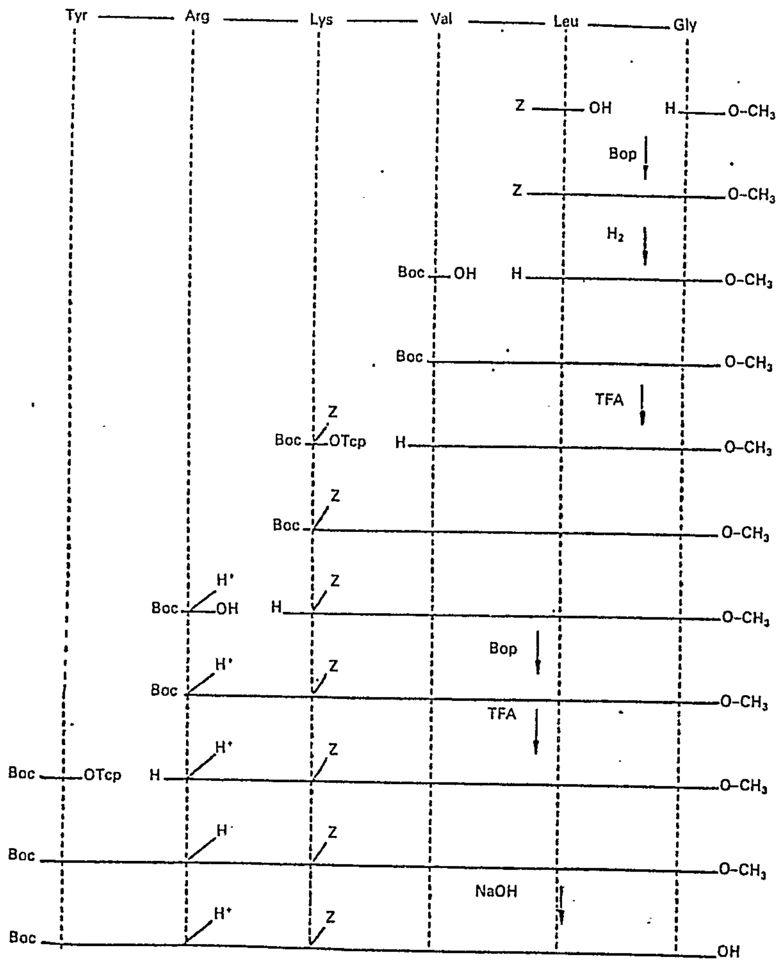


Tableau IX (fragment $H_1$)

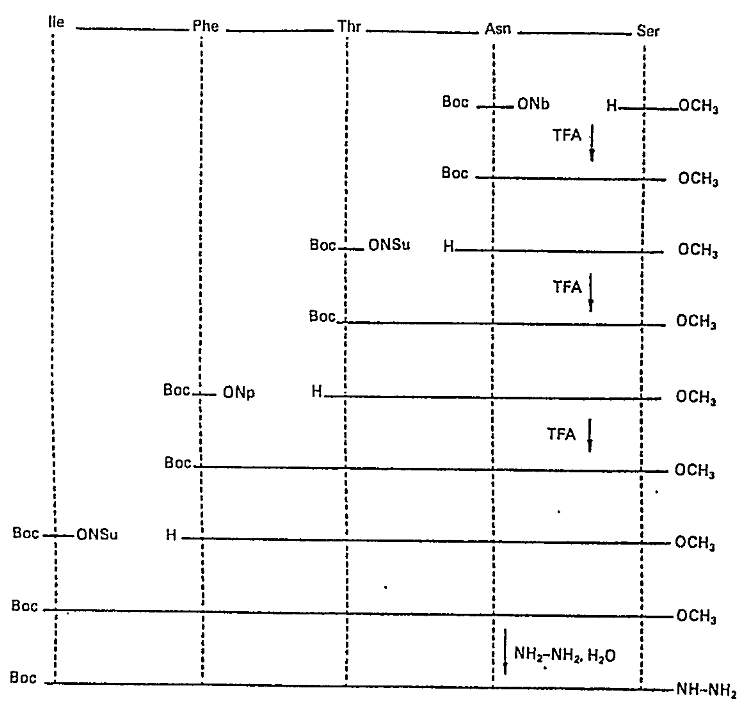

Tableau X (fragment I)

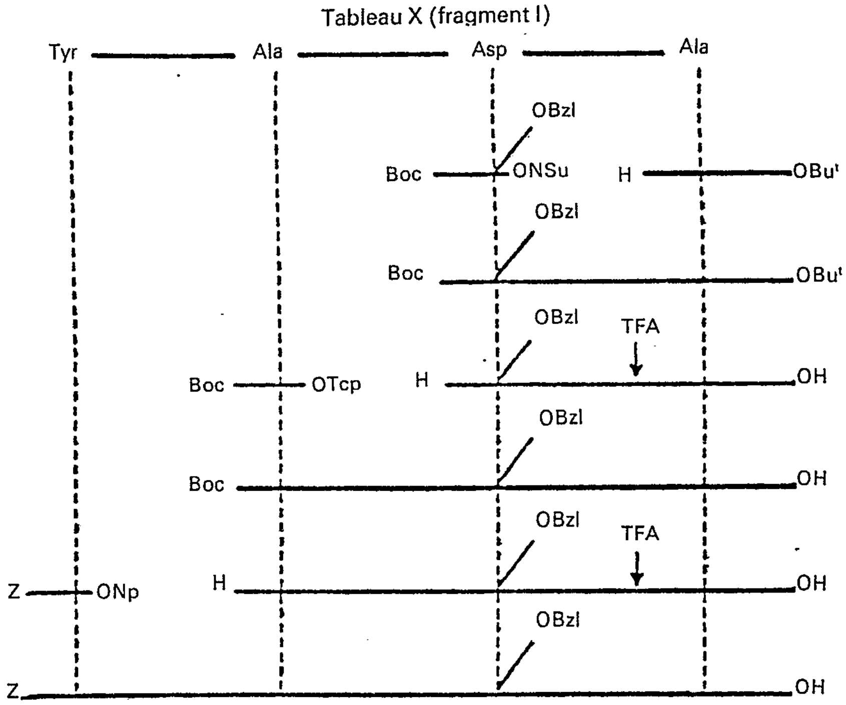


Synthese du peptide $K_1$ (hGRF 20–44)

OBz1 H+ H+ H+
Boc-Gln-Glu-Arg-Gly-OH + H-Ala-Arg-Ala-Arg-Leu-$NH_2$ —BOP→
[B'1] [A] puis H+

OBz1 H+ H+ H+
H-Gln-Glu-Arg-Gly-Ala-Arg-Ala-Arg-Leu-$NH_2$

OBz1
BOP Boc-Glu-Ser-Asn-OH
puis H+ [B'2]

OBz1 OBz1 H+ H+ H+
H-Glu-Ser-Asn-Gln-Glu-Arg-Gly-Ala-Arg-Ala-Arg-Leu-$NH_2$

BOP H+
puis H+ Boc-Ser-Arg-Gln-Gln-Gly-OH

H+ OBz1 [C] OBz1 H+ H+
H-Ser-Arg-Gln-Gln-Gly-Glu-Ser-Asn-Gln-Glu-Arg-Gly-Ala-Arg-Ala-Arg-Leu-$NH_2$

Curtius OBz1
puis H+ Boc-Leu-Gln-Asp-Ile-Met-$N_3$

OBz1 H+ [D'] OBz1 OBz1
H-Leu-Gln-Asp-Ile-Met-Ser-Arg-Gln-Gln-Gly- Glu-Ser-Asn-Gln-Glu-Arg

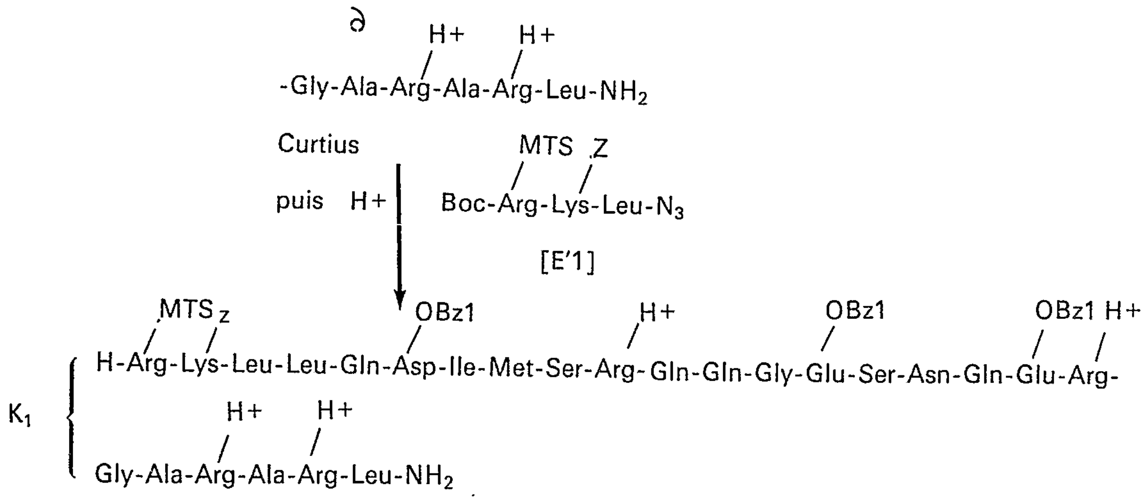


Schema I

Synthese du peptide J hGRF (5–19)

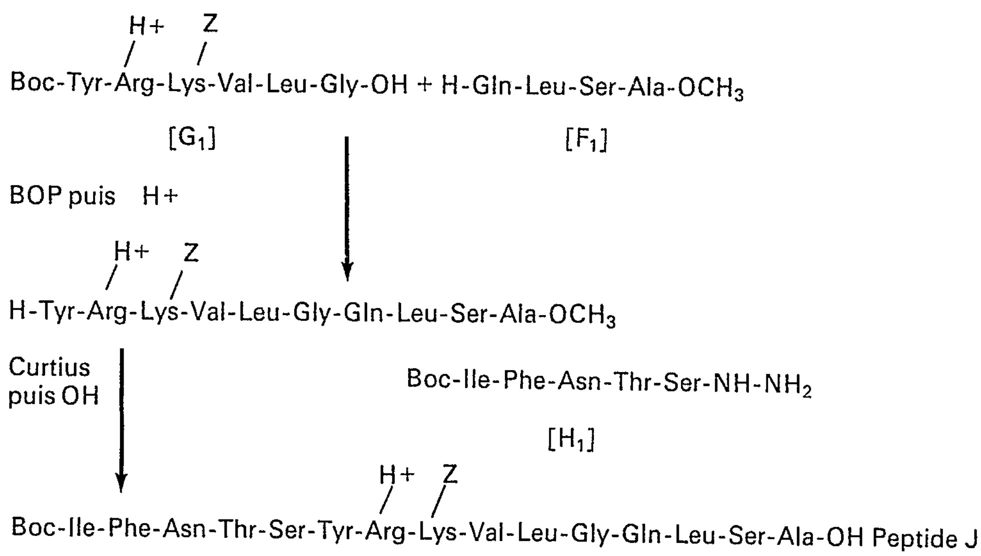


Schema II

Passage à la séquence du GRF 1–44

Peptide J + peptide $K_1$

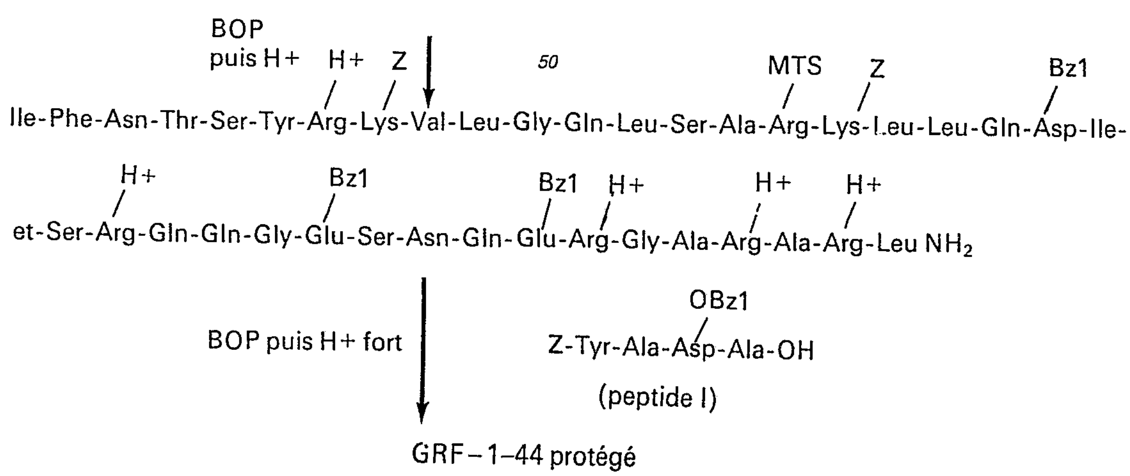


Schema III

Les exemples suivants permettront de mieux comprendre la portée de l'invention.

Les abréviations suivantes seront utilisées.

Les acides aminés sont représentés par les symboles recommandés par la commission de nomenclature de l'IUPAC-IUB section biochimie.

| | |
|---|---|
| Ala: | Alanine |
| Arg: | Arginine |
| Asn: | Asparagine |
| Asp: | Acide aspartique |
| Gln: | Glutamine |
| Glu: | Acide glutamique |
| Gly: | Glycine |
| Ile: | Isoleucine |
| Leu: | Leucine |
| Lys: | Lysine |
| Met: | Méthionine |
| Phe: | Phénylalanine |
| Ser: | Sérine |
| Thr: | Thréonine |
| Tyr: | Tyrosine |
| Val: | Valine |

A l'exception de la glycine, ils ont tous la configuration L.

HPLC: chromatographie liquide haute performance
CCM: chromatographie sur couches minces

OBzl: ester benzylique

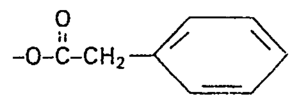

Z: benzyloxycarbonyle (carbamate)

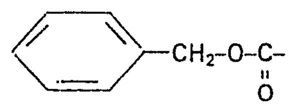

Boc: tertiobutyloxycarbonyl (carbamate)

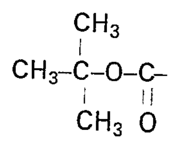

DMF: diméthylformamide
NEM: N-éthylmorpholine
TFA: acide trifluoroacétique
MA: méthode de couplage aux anhydrides mixtes
AcOEt: acétate d'éthyle
OMe: $OCH_3$
ONSu: ester activé avec la N-hydroxy succinimide

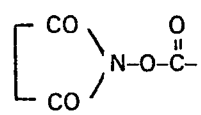

ONp: ester activé avec l'ortho nitrophénol

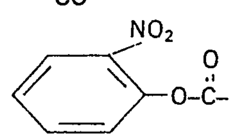

Troc: trichloroéthoxycarbonyle (carbamate)

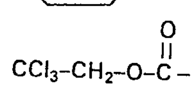

$Et_2O$: éther éthylique

OBu[t]: ester avec le tertiobutanol

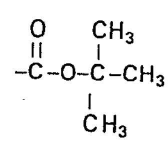

MTS: 2,4,6-triméthyl-benzène-sulfonyle (mésityl-sulfonyle)
OTcp: ester activé avec le 2,3,5-trichlorophénol
OHBT: N-hydroxybenzotriazole

ONb: ester activé avec le N-hydroxy norbornène-5 dicarboximide-2,3

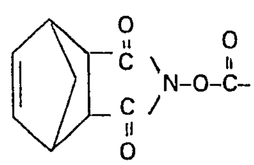

NbOH: N-hydroxy-norbornène-5 dicarboximide-2,3

BOP: hexafluorophosphate de benzotriazolyloxyphosphonium

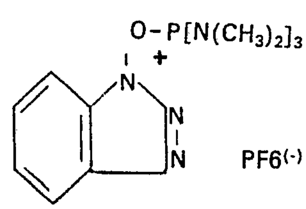

| | |
|---|---|
| DCHa: | dicyclohexylamine |
| DCU: | dicyclohexylurée |
| DCC ou: DCCi | dicyclohexylcarbodiimide |
| TA: | température ambiante |
| DIPEA: | diisopropyléthylamine |
| EPP: | pureté polypeptidique |
| TFMSA: | acide trifluorométhane sulfonique |
| AAA: | analyse d'amino acides |

Milieux de chromatographie exprimés en volumes:

| | |
|---|---|
| $BEW_1$: | butanol, AcOH, $H_2O$ 72/7/21 |
| $BEW_2$: | butanol, AcOH, $H_2O$ 67/10/23 |
| $BPEW_1$: | butanol, pyridine, AcOH, $H_2O$ 50/12/12/25 |
| EPAW: | AcOEt, pyridine, $HCO_2H$, $H_2O$ 63/21/10/6 |
| $BPEW_2$: | butanol, pyridine, AcOH, $H_2O$ 42/24/4/30 |

Exemple I

Synthèse de H – Ala – Arg – Ala – Arg – Leu – $NH_2$ (fragment A)

$$\begin{array}{l}\quad NO_2\\ \quad\ \vert\\ 1.\ H\text{–}Arg\text{–}Leu\text{–}NH_2\end{array}$$

Dissoudre à la température ambiante 130 g de leucine amide (H–Leu–$NH_2$) dans 1,5 l de DMF. Ajouter 333 g de Boc–Arg ($NO_2$)–OH puis 500 g de BOP. Ajuster le pH à 7 au papier pH (sur de petits prélèvements dilués à l'eau) et à l'aide de N-éthylmorpholine (NEM). Le milieu est agité et on suit l'évolution de la réaction par CCM. La réaction est terminée au bout de 4 h. Le milieu est évaporé à sec sous vide à 25 °C. Le résidu est repris par 1 litre d'eau et on obtient un solide qui est lavé à l'eau, puis avec une solution aqueuse de $NaHCO_3$ à 5% à l'eau, à l'acétate d'éthyle et, finalement, on sèche le solide à l'air. On contrôle par CCM.

Le solide précédent est introduit dans 2 l d'un mélange 50–50 en volume d'acide trifluoroacétique-chlorure de méthylène. Le milieu est agité 10 min à la température ambiante et évaporé à sec sous vide à la température ambiante. Le résidu d'évaporation est repris dans l'éther, essoré, séché et contrôlé par CCM et RMN. Rendement: 339 g (90%) en trifluoroacétate d'un solide blanc.

2. H–Ala–Arg($NO_2$)Leu–$NH_2$

443 g de trifluoroacétate de H–Arg($NO_2$)Leu–$NH_2$ sont dissous dans 2 l de DMF. On ajoute 200 g de Boc–Ala–OH et ensuite 500 g de BOP. On ajuste le pH à 7 au papier pH (sur de petits prélèvements du milieu réactionnel) à l'aide de NEM. Le milieu est agité et l'évolution de la réaction est suivie par CCM. La réaction est terminée au bout de 4 h. Le milieu est évaporé à sec sous vide à 25 °C. Le résidu est repris par 2 l d'eau et 2 l d'acétate d'éthyle. La phase organique est lavée par une solution aqueuse de $NaHCO_3$ à 5%, à l'eau, séchée et évaporée. Le tripeptide est recristallisé dans l'acétate d'éthyle-éther et finalement séché sous vide. Il est contrôlé par CCM et RMN.

Le précédent produit séché est traité par un mélange 50–50 (volume) de TFA–$CH_2Cl_2$ dans les conditions de l'exemple précédent (I-1). L'isolement est effectué également dans les mêmes conditions.

Rendement: 412 g (80%) exprimé en trifluoroacétate d'un produit pulvérulent blanc (contrôlé en CCM et RMN).

3. Arg($NO_2$)–Ala–Arg($NO_2$)–Leu–$NH_2$

A partir de 515 g de H–Ala–Arg($NO_2$)Leu–$NH_2$ dans 2,5 l de DMF et 333 g de Boc–Arg ($NO_2$)–OH et 500 g de BOP, on obtient en utilisant les conditions opératoires décrites dans l'exemple I, 1, après traitement au mélange TFA–$CH_2Cl_2$, 607 g (85%) d'un solide blanc contrôlé par CCM et RMN.

4. Z–Ala–Arg($NO_2$)–Ala–Arg–Leu–$NH_2$

A partir de 715 g de trifluoroacétate de H–Arg ($NO_2$)Ala–Arg($NO_2$)–Leu–$NH_2$ en solution dans 4 l de DMF et 233 g de Z–Ala–OH et 500 g de BOP en utilisant la même technique que celle décrite en I-2 (et sans traitement dans ce cas par TFA–$CH_2Cl_2$), on obtient après recristallisation dans le mélange DMF-éther,12 g de Z–Ala–Arg ($NO_2$)–Ala–Arg($NO_2$)Leu–$NH_2$ (76%) sous forme d'un solide pulvérulent blanc, contrôlé par CCM et RMN.

5. Ala–Arg–Ala–Arg–Leu–$NH_2$, 3HCl

200 g de Z–Ala–Arg($NO_2$)Ala–Arg($NO_2$)–Leu–$NH_2$ (0,25 mole) sont mis en suspension dans 2 l de méthanol contenant 0,8 mole de HCl. On ajoute 40 g de Pd/C à 10% de Pd, et le milieu est agité sous atmosphère d'hydrogène sous 1,2 bar de pression, pendant 24 h. Après cet intervalle de temps, on contrôle la fin de la réaction par CCM. Le catalyseur est éliminé par filtration et le solvant évaporé sous vide à la température ambiante.

Le résidu solide est purifié par chromatographie sur gel de silice en utilisant comme milieu d'élution le mélange butanol, pyridine, $HO_2CCH_3$,$OH_2$ (50–12–12–25 en volume).

Les fractions contenant le produit pur sont réunies, évaporées et lyophilisées.

Rendement: 119 g (69%) d'un solide pulvérulent blanc.

Contrôles: RMN, CCM.

Analyse d'aminoacides: Leu 1,03 (1)
Arg 1,92 (2)
Ala 1,95 (2)

Exemple II

synthèse de Boc–Gln–Glu(Obzl)–Arg–Gly–OH, HCl (fragment B'[1])

1. Z–Arg($NO_2$)–Gly–OBzl

On dissout 3,37 g (0,01 mole) de tosylate de H–Gly–OBz1 dans 15 ml de DMF, puis on ajoute 1 équivalent de NEM (1,3 ml). Cette solution est ajoutée à une solution de 3,53 g (0,01 mole) de Z–Arg($NO_2$)–OH dans 15 ml de DMF contenant un équivalent de NEM (1,3 ml). On ajoute alors 4,5 g (0,01 mole) de BOP au mélange réactionnel dont le pH est ensuite amené à 7 par addition de NEM et qui est agité à TA pendant 2 h 30 (la fin de la réaction est déterminée par CCM: chloroforme-méthanol (3/1). Le mélange réactionnel est évaporé à sec sous pression réduite (0,1 mm de Hg soit 13,33 Pa) à température inférieure à 30 °C. Le résidu est repris dans 100 ml d'acétate d'éthyle. Cette solution est versée dans 120 ml d'eau glacée vigoureusement agitée. Après quelques instants d'agitation, un précipité se forme qui est abandonné une nuit au réfrigérateur. Le solide est essoré et lavé successivement à l'état solide avec:

– 2 × 100 ml d'une solution aqueuse de bicarbonate de soude
– 2 × 100 ml d'une solution aqueuse de $SO_4HK$–$SO_4K_2$ à 5%
– 2 × 100 ml d'eau
– 2 × 100 ml d'éther

puis séché sous vide (13,33 Pa) jusqu'à poids constant.

Rendement: 4 g (80%).
Point de fusion Koffler: 149 °C
CCM: chloroforme-méthanol (3/1) Rf: 0,73
Contrôle: RMN

2. HCl–H–Arg–Gly–OH

On met en suspension 100 g (0,2 mole) de Z–Arg($NO_2$)–Gly–OBz1 dans un mélange de 600 ml d'eau, 600 ml d'acide chlorhydrique N et 100 ml de tétrahydrofuranne. On ajoute 60 g de Pd/C à 10% contenant 50% d'humidité. Ce mélange est hydrogéné pendant 48 h à température ambiante et sous une pression de 2 kPa.

Le catalyseur est ensuite filtré et la solution aqueuse est amenée à pH 6,5 par addition de résine Amberlite IR 45 (forme OH). La résine est essorée. La solution est ensuite amenée à pH 8,5 par addition d'une nouvelle quantité de résine et l'ensemble est agité pendant 20 min au rotavapor sous vide (3,33 kPa). On essore la résine et on vérifie l'absence de chlorure d'ammonium dans la solution aqueuse par le test de Nesler, puis on évapore à sec cette solution sous pression réduite (13,33 Pa) à température inférieure à 30 °C. On obtient un résidu gommeux qui est séché une nuit dans un dessiccateur avec de l'anhydride phosphorique.

Rendement: 43,19 g (80,6%).
CCM: acétate d'éthyle-pyridine-acide formique-eau 40–21–10–6
Rf: 0,1 test de SaKaguchi positif (spot rouge)
Contrôle RMN

3. Boc–Glu(OBz1)–Arg(HCl)–Gly–OH

On dissout 41,5 g de HCl–H–Arg–Gly–OH (0,155 mole) dans un mélange de 400 ml de DMF et de 130 ml d'eau. Le pH est ajusté à 7 par addition d'acide chlorhydrique N. A cette solution, on ajoute simultanément:

– une solution de 68,8 g de Boc–Glu(OBz1)–ONP (0,150 ml) dans un mélange de 100 ml de DMF et de 40 ml d'eau
– une solution de 22,7 g d'hydroxybenzothiazole (0,150 mole) dans un mélange de 100 ml de DMF et de 40 ml d'eau
– une solution de 37,8 ml de NEM (2 × 0,150 mole) dans 100 ml de DMF.

Le mélange réactionnel est agité à TA et la réaction est suivie en CCM (chloroforme-méthanol-acide acétique 95–5–9 et pyridine-acétate d'éthyle-acide formique-eau 21–40–10–6).

Au bout de 1 h de réaction, on rajoute 13,76 g de Boc–Glu(OBz1)–ONP.

Au bout de 2 h de réaction, on rajoute 13,76 g de Boc–Glu(OBz1)–ONP.

Au bout de 3 h de réaction, on rajoute 7 g de Boc-Glu(OBz1)–ONP et on poursuit la réaction pendant 1 h.

Le mélange réactionnel est alors évaporé à sec sous pression réduite (13,33 Pa) et à température inférieure à 30 °C. On obtient une huile épaisse qui est dissoute dans 500 ml d'acétate d'éthyle. Par addition de 1 litre d'éther, une huile épaisse se forme. On abandonne au repos 24 h au réfrigérateur, puis on décante la phase liquide. L'huile résiduelle est reprise dans 500 ml de méthanol. A cette solution on ajoute 500 g de silice (70–230 mesh soit 62–210 μm), et on évapore le solvant au rotavapor. La poudre obtenue est mélangée avec un mélange chloroforme-méthanol 80–20 et le gel formé est introduit au sommet d'une colonne de gel de silice (hauteur 200 cm, diamètre 85 mm) montée dans le mélange chloroforme-méthanol 80–20. Le produit est élué avec:

– mélange de chloroforme-méthanol 80–20: 25 litres
– mélange de chloroforme-méthanol (50–50): 10 litres
– méthanol: 30 litres.

La purification est suivie par CCM (pyridine-acétate d'éthyle-acide formique-eau 21–40–10–6). On recueille 2 fractions:

– fraction A 29,80 g
CCM: pyridine-acétate d'éthyle-acide formique-eau 21–40–10–6
Rf: 0,75
Contrôle: RMN
HPLC – EPP: 96,98%
AAA: Glu 1,01–Gly 1,01–Arg 0,98
– fraction B 36,82 g
CCM: pyridine-acétate d'éthyle-acide formique-eau 21–40–10–6

Rf: 0,75
$^1$H RMN spectre conforme
Contrôle: HPLC – EPP: 99,61%
AAA: Glu 0,93–Gly 0,99–Arg 1,08.

4. Boc–Gln–Glu(OBz1)–Arg(HCl)–Gly–OH

52,78 g (0,09 mole) du précédent produit sont mis en suspension dans 300 ml de chlorure de méthylène. On ajoute à la température ambiante 300 ml de TFA. Le milieu est conservé 1 h à cette température et finalement le solvant et l'excès de TFA sont évaporés sous vide au rotavapor sans chauffage. Le résidu d'évaporation est repris dans l'éther (300 ml). Il précipite en donnant un produit pulvérulent blanc. Il est soigneusement lavé à l'éther et séché sous vide dans un dessiccateur. Le rendement est quantitatif et le produit est utilisé tel quel pour la mise en place de la glutamine. On le dissout dans 300 ml de DMF. On ajoute 60 ml d'eau et on ramène le pH à 7 par le NEM (contrôles effectués sur de petits prélèvements). 36,39 g de Boc–Gln–ONp (0,1 mole) sont dissous à part dans 150 ml de DMF. On dissout également à part 22,7 ml de NEM (0,2 mole) dans 150 ml de DMF et 15,4 g de HOBT (0,1 mole) dans 150 ml de DMF. Ces 3 dernières solutions sont ajoutées simultanément pendant 1 h à la première solution du peptide dans le DMF/$H_2O$ en s'assurant que le pH se maintient entre 6 et 7. Le milieu est agité à la température ambiante pendant 4 heures et évaporé à sec sous vide à la température ambiante. Le résidu d'évaporation est repris 3 fois par 150 ml d'éther. L'huile obtenue est dissoute dans la phase supérieure du mélange butanol–$H_2O$ à 50%. On lave 2 fois la phase supérieure avec 200 ml de la phase inférieure. Les phases inférieures sont extraites avec 50 ml de phase supérieure. Les phases supérieures réunies sont évaporées à sec sous vide. Le résidu d'évaporation est repris dans 150 ml d'isopropanol chaud et le produit est précipité par addition d'éther. On obtient 61,13 g (94,9%) d'un produit caractérisé par:

AAA: Gln(Glu): 2,01 – Gly: 1,01 – Arg: 0,98 –
HPLC: EPP = 96,5%
$^1$H RMN: conforme
CCM (EPAW) Rf = 0,58

Exemple III
Boc–Glu(OBz1)–Ser–Asn–OH (fragment B'$_2$)

1. Boc–Ser–Asn–OBz1

17,65 g (0,0525 mole) du sel de TFA de H–Asn–OBz1 sont dissous dans 100 ml de DMF. Le pH est amené à 7 par addition de NEM (5,5 ml environ). On ajoute ensuite 19,2 g (0,0525 mole) de Boc–Ser–ONb. Le pH est ramené à 7 par addition de NEM. Le mélange est agité à la température ambiante avec contrôles intermittents de pH. Au bout de 4 h la réaction est terminée (CCM). On évapore à sec sous vide le milieu réactionnel en maintenant à une température inférieure à 30 °C. La résidu d'évaporation est dissous dans 150 ml d'acétale d'étyle et la solution obtenue lavée successivement avec 2 × 120 ml d'une solution aqueuse saturée de NaCl, 3 × 120 ml d'une solution aqueuse saturée de $NaHCO_3$, 3 × 120 ml d'une solution aqueuse de $KHSO_4$–$K_2SO_4$ et finalement 1 × 120 ml d'une solution aqueuse saturée en NaCl. La phase organique séchée sur $MgSO_4$ anhydre est évaporée à sec sous vide. Lerésidu est repris dans l'éther où il précipite pour donner une poudre blanche qui est séchée sous vide.

Rendement: 16,64 g (78%)
$^1$H RMN: conforme
CCM–: $CHCl_3$–méthanol 3–1 – Rf: 0,6 –

2. Ser–Asn–OBz1,TFA

143,5 g (0,35 mole) du produit préparé selon la technique ex. III no. 1 sont dissous dans 700 ml de TFA. Un léger insoluble est filtré et on conserve le milieu 30 min à la température ambiante. Le milieu réactionnel est ensuite évaporé à sec sous vide à la température ambiante. Le résidu est repris par 50 ml d'éther et évaporé à nouveau. Le résidu s'organise en une poudre blanche dans l'hexane-éther. Le rendement est quantitatif – CCM: $ClCl_3$-méthanol 3–1 – Rf: 0,1.

3. Ser–Asn–OH, TFA

116 g (0,35 mole) du précédent produit sont dissous dans 1,6 l d'eau. On ajoute 35 g de Pd/C à 10% en Pd et à 50% d'humidité. Le milieu est agité 24 h à la température ambiante sous atmosphère d'hydrogène. Après cet intervalle de temps, on recharge avec 20 g du même catalyseur et poursuit la réaction 24 h de plus. Le catalyseur est éliminé par filtration et l'eau évaporée sous vide à 30 °C. Le résidu est repris par 200 ml de méthanol et évaporé pour finir d'éliminer l'eau. Après cette dernière évaporation, on reprend par l'éther et un solide précipite dans ces conditions. Il est essoré et séché sous vide.

Rendement quantitatif: CCM: EPAW – Rf: 0,13.

4. Boc–Glu–Ser–Asn–OH

0,35 mole du précédent produit (environ 117 g) sont dissous dans 300 ml d'eau. On ajoute ensuite 0,7 mole de triéthylamine. Le pH est alors de 9 et on dilue avec 1,5 l de DMF. Ensuite, on introduit rapidement 0,35 mole (152 g) de Boc–Glu(OBz1)-ONSu en solution dans 200 ml de DMF. Le pH est maintenu à 7,2 par addition de triéthylamine. La réaction est terminée au bout de 3 h. Le milieu est évaporé à sec sous vide à 30 °C. Le résidu est repris dans 3 fois 1 l d'éther et finalement dissous dans 1,5 l de la phase supérieure du mélange butanol-eau 50–50. On lave par 600 ml d'une solution à 5% de $KHSO_4$–$K_2SO_4$ puis 3 fois par 300 ml de phase inférieure du même mélange butanol-eau et, enfin, par 300 ml d'une solution saturée en NaCl. On sèche sur $MgSO_4$ anhydre et on évapore à sec. Le résidu précipite en donnant une poudre blanche dans l'éther et l'hexane. Après séchage sous vide, on obtient 120 g (63,8%) d'un produit pulvérulent blanc.

$^1$H RMN conforme –
AAA: Asp: 1,02 – Ser: 0,90 – Glu: 1,09 –
CCM (PBEW$_1$) – Rf: 0,55 –
EPP: 96%

Exemple IV
synthèse de Boc–Ser–Arg–Gln–Gln–Gly–OH,HCl (fragment C)

1. Z–Gln–Gly–OMe

Une suspension refroidie sur bain de glace de 75,36 g de HCl, H–Gly–OMe (0,6 M) dans 1 litre de DMF est additionnée de 168,2 g de Z–Gln–OH (0,6 M), de 256,41 g de BOP (0,7 M) et de 165,4 ml de N-éthylmorpholine (1,3 M). Après 20 h de réaction à TA, la solution est concentrée sous pression réduite et le résidu repris avec de l'acétate d'éthyle. La solution organique est lavée par:

- une solution de bicarbonate de sodium
- une solution de chlorure de sodium
- une solution de $KHSO_4/K_2SO_4$
- une solution de chlorure de sodium

La solution organique est séchée sur $MgSO_4$ et filtrée. Après 20 h de repos, il y a cristallisation dans chacune des solutions. Après filtration, les fractions provenant d'une cristallisation en milieu aqueux sont regroupées et lavées dans AcOEt saturé en eau pour donner après filtration et séchage sous vide 7,78 g de produit (F. 158–159 °C). La fraction provenant de la cristallisation dans AcOEt est lavée dans AcOEt saturé en eau pour donner, après filtration et séchage sous vide 61,8 g de produit (F. 150–154 °C). La phase aqueuse et la phase organique donnent une cristallisation supplémentaire. Ces deux derniers produits sont regroupés et lavés comme précédemment pour fournir 38,05 g supplémentaires de qualité comparable.

Rendement global: 107,63 g
Contrôles RMN et CCM

2. HCl, H–Gln–Gly–OMe

Une solution de 61,8 g de Z–Gln–Gly–OMe (175,9 mM) dans 704 ml de DMF, 880 ml de méthanol est additionnée sous refroidissement par bain de glace de 176 ml d'une solution d'HCl N, puis de 3,1 g de Pd/C à 10%. Après 3 h 30 d'hydrogénation sous surpression de 46,6 kPa, la catalyseur est filtré sur célite et la solution est concentrée sous pression réduite. Le résidu est utilisé directement dans l'étape suivante.

3. Boc–Gln–Gln–Gly–OMe

Une solution du résidu obtenu à l'étape précédente (théoriquement 175,9 mM) dans 500 ml de DMF, refroidie sur bain de glace est additionnée de 77,6 g de Boc–Gln–ONp (211,1 mM), 26,4 g d'hydroxybenzotiazole (211,1 mM) et de N-éthylmorpholine jusqu'à obtenir un pH de 7. Après 20 h de réaction à TA, la solution est prise en masse. Par addition d'acétate d'éthyle, on obtient un précipité qui est essoré, lavé à AcOEt et séché sous vide. On obtient 64,27 g de produit, soit un rendement de 82%.
Contrôles RMN et CCM

4. TFA, H–Gln–Gln–Gly–OMe

Une suspension refroidie sur bain de glace de 98,83 g de Boc–Gln–Gln–Gly–OMe (221,8 mM) dans 300 ml de dichlorométhane est additionnée de 400 ml de TFA. Après 30 min de réaction sur bain de glace et 1 h à TA, la solution est concentrée sous pression réduite jusqu'à la moitié de son volume initial et le résidu est versé sur de l'éther en agitation. Après filtration, lavage et séchage sous vide, on obtient 119 g de produit.

5. Boc–Arg($NO_2$)–Gln–Gln–Gly–OMe

Une solution des 119 g du produit précédent dans 1190 ml de DMF refroidie sur bain de glace est additionnée de 77,91 g de Boc–Arg-($NO_2$)–OH (244 mM), de 97,51 g de BOP (266,2 mM) et de N-éthylmorpholine jusqu'à obtention de pH 7. Après 20 h de réaction à TA, la solution est versée sur 8 l d'acétate d'éthyle. Le précipité obtenu est filtré, lavé à AcOEt, puis séché sous vide. On obtient 153,7 g de produit.
Contrôles RMN et CCM.

6. TFA, H–Arg($NO_2$)–Gln–Gln–Gly–OMe

Une suspension refroidie sur bain de glace de 153,7 g de Boc–Arg($NO_2$)–Gln–Gln–Gly–OMe (théoriquement 221,8 mM) dans 600 ml de dichlorométhane est additionnée de 750 ml de TFA. Après 1 h à TA, on ajoute encore 250 ml de TFA et, 30 min plus tard, 250 ml de TFA. Après 1 h supplémentaire de réaction, on concentre la solution jusqu'au tiers du volume initial et verse le résidu sur 3 l d'éther en agitation. Le précipité formé est essoré, lavé à l'éther et séché sous vide. On obtient 167 g de produit.

7. Boc–Ser–Arg($NO_2$)–Gln–Gln–Gly–OMe

Une solution refroidie sur bain de glace de 167 g de TFA, H–Arg($NO_2$)–Gln–Gln–Gly–OMe (théoriquement 221,8 mM) dans 1,5 l de DMF est additionnée de 97,52 g de Boc–Ser–ONb (266,2 mM), 33,31 g d'hydroxybenzotriazole (266,2 mM) et de N-éthylmorpholine jusqu'à obtenir un pH de 7. Après 3 h de réaction à TA, on évapore une partie du DMF et l'on verse la solution résiduelle sur de l'acétate d'éthyle sous agitation. Le précipité est filtré, lavé à l'acétate d'éthyle et séché sous vide.

On obtient 153,3 g de produit, soit un rendement de 94,2% sur 4 étapes.
Contrôles RMN et CCM.

8. Boc–Ser–Arg–Gln–Gln–Gly–OMe, AcOH

Une solution de 153,3 g de Boc–Ser–Arg ($NO_2$)–Gln–Gln–Gly–OMe (208,9 mM) dans 1 litre de méthanol, 1 litre d'eau et 500 ml d'acide acétique est hydrogénée pendant 20 h en présence de 10 g de Pd/C à 10%. Après filtration du catalyseur et concentration de la solution, le résidu repris à l'eau est lyophilisé. On obtient 153 g de produit, soit 97,8% de rendement.

9. Boc–Ser–Arg–Gln–Gln–Gly–OH, HCl

Une solution de 104,4 g de Boc–Ser–Arg–Gln–Gln–Gly–OMe (139,4 mM) dans 2 l de DMF est additionnée sous refroidissement par bain de glace de 1 litre d'eau, puis de 27,88 g de NaOH (697 mM) dans 50 ml d'eau. Après 15 min de

réaction à température voisine de 15 °C, on neutralise par une solution d'acide chlorhydrique N jusqu'à obtenir un pH de 6,5. Après évaporation, on triture le résidu dans de l'acétate d'éthyle. Le précipité formé est essoré, lavé à AcOEt et séché sous vide, puis à l'air.

On obtient 132,7 g de produit.

Purification: 109 g de Boc–Ser–Arg–Gln–Gln–Gly–OH, HCl sont purifiés par distribution à contre-courant dans le mélange n-butanol-m éthanol-eau (4–1–5).

Après 700 transferts, on fractionne en trois parties qui, après évaporation et lyophilisation, donnent:

- une fraction de 27,5 g et
- deux fractions à repurifier de 23,79 g et 16,90 g

Contrôles RMN et CCM

AAA:

| | |
|---|---|
| Ser: | 0,91 |
| Arg: | 0,97 |
| Gln(Glu): | 2,05 |
| Gly: | 1,01 |

Exemple V

Boc–Leu–Gln–Asp–Ile–Met–NH–$NH_2$ (fragment D')

1. Boc–Met–NH–NH–Troc

4,31 g (10 mM) de Boc–Met–OH, DCHa en solution dans 50 ml acétate d'éthyle sont traités en présence de 20 ml d'eau par une solution saturée de bisulfate de potassium jusqu'à pH = 3; la phase aqueuse est extraite plusieurs fois à l'acétate d'éthyle et les extraits séchés sur sulfate de magnésium. A cette solution, on ajoute 2,28 g (11 mM) de $H_2N$–NH–Troc (Troc = trichloro–2,2,2, éthoxycarbonyle, ce réactif étant préparé selon YAJIMA, Chem. Pharm. Bull. 1971, 19, 420).

Après refroidissement au bain de glace, on ajoute 2,37 g (11 mM) de DCC à 97% en solution dans 10 ml d'acétate d'éthyle. Après une nuit pendant laquelle le produit revient progressivement à TA, la dicyclohexylurée est filtrée et séchée (2,04 g) et la solution organique est lavée successivement par les solutions aqueuses suivantes: sulfate-bisulfate 5% 2 fois C1 Na 2 M 2X bicarbonate de sodium 5%, 2 fois C1 Na M 2 fois et enfin à l'eau 2 fois. Après séchage sur sulfate de magnésium et concentration du solvant presque à sec, on ajoute de l'hexane jusqu'à début de trouble et garde à +4° une nuit après quoi le précipité formé est filtré, lavé par un mélange d'hexane et d'acétate d'éthyle 4/1 et séché sous vide, on obtient ainsi 3,46 g (79%) Fc = 92–4° alpha D 25% = 32° – C = 1, dioxane.

CCM dans chloroforme-méthanol–AcOH 95/5/3 – Rf = 0,45

2. TFA, H–Met–NH–NH–Troc

43,9 g (0,1 M) de Boc–Met–NH–NH–Troc en solution dans 200 ml de dichlorométhane et 20 ml d'éthanedithiol sont traités au bain de glace sous azote et avec agitation par 200 ml d'acide trifluoroacétique, on ôte le bain froid et laisse sous agitation pendant une heure. Le produit est isolé par élimination des réactifs volatils d'abord sous 2,6 kPa puis 13,33 Pa de pression, l'huile résiduelle est reprise 2 fois par 75 ml d'isopropanol en évaporant sous vide puis lavée 2 fois par 75 ml d'hexane par décantation après quoi elle est séchée sous vide en présence de potasse une nuit, après quoi elle commence à se solidifier et est utilisée telle quelle dans l'opération suivante.

3. Boc–Ile–Met–NH–NH–Troc

L'huile ci-dessus (environ 0,1 M) de TFA, H–Met–HN–NH–Troc en solution dans 6500 ml d'acétate d'éthyle et refroidie au bain de glace est traitée par 12,7 ml (0,1 M) de N-éthylmorpholine puis 14,85 g (0,1 M) d'HOBt, 1 h 20 puis par 34,35 g (0,08 M) de Boc–Ile–OSu suivis de 12,7 ml (0,1 M) de N-éthylmorpholine (NEM). Après 1/2 h, le bain froid est ôté et par la suite de la NEM est ajoutée périodiquement de manière à maintenir le pH apparent vers 7.

Après 20 h, la réaction est complète et l'isolement se fait par lavages successifs par les solutions aqueuses suivantes: sulfate-bisulfate de potassium 5% 3 fois, de l'eau 3 fois, du bicarbonate de sodium 5% 3 fois et enfin, à l'eau jusqu'à neutralité.

Après séchage sur sulfate de magnésium et évaporation du solvant sous vide, on obtient 64 g de gomme qui est chromatographiée sur une colonne de silice avec du dichlorométhane contenant de 0 à 1,5% de méthanol. On obtient ainsi 30 g (68%) de produit ayant une pureté HPLC de 98,9% et un spectre RMN en accord avec la structure attendue. Fc: 88–92 °C.

4. TFA, H–Ile–Met–NH–NH–Troc

27,6 g (50 mM) de Boc–Ile–Met–NH–NH–Troc en solution dans 140 ml de dichlorométhane et 14 ml d'éthanedithiol sont refroidis au bain de glace et agités sous azote puis on leur ajoute en 5–6 min, 140 ml d'acide trifluoroacétique. Le bain froid est ôté et, après 45 min, le produit est isolé par évaporation au maximum des réactifs, reprise de l'huile résiduelle par 2 × 60 ml d'isopropanol suivie d'évaporation et enfin par 2 lavages par 60 ml de pentane. Après séchage une nuit sur potasse sous vide, on obtient une huile d'aspect vitreux qui est utilisée dans l'opération suivante (30 g).

5. Boc–Asp(OBz1)–Ile–Met–NH–NH–Troc

Les 30 g d'huile précédente en solution dans 250 ml de THF sont traités par 6,4 ml (50 mM) de NEM et 18,9 (45 mM) de Boc–Asp(OBz1)ONSu. Le pH apparent est ajusté entre 6 et 7 par additions successives de NEM. Après 4 h, le produit est isolé par évaporation du THF, reprise dans 400 ml d'acétate d'éthyle, suivie des mêmes lavages que l'homologue précédent (3). Le résidu, après séchage et évaporation (36,5 g), est purifié par chromatographie sur une colonne de silice avec du dichlorométhane comme solvant contenant de 0 à 1,5% de méthanol. On obtient ainsi

25 g (66%) de produit présentant un spectre RMN conforme à la structure attendue. Fc = 96–100 °C.

6. Boc–Gln–Asp(OBz1)–Ile–Met–NH–NH–Troc

42 g (55 mM) de Boc–Asp(OBz1)–Ile–Met–NH–NH–Troc sont dissous dans un mélange de 300 ml de chlorure de méthylène anhydre et 30 ml d'éthane-dithiol, puis traités en 15–20 min par 300 ml de TFA à TA sous agitation. 45 min après la fin de l'addition, le mélange est évaporé à sec, repris plusieurs fois dans l'éther anhydre et décanté, puis séché sous vide en présence de potasse. Le sel de TFA ci-dessus est dissous dans 270 ml de DMF et refroidi à +5 – +10 °C. Le pH est amené à 7 (papier pH) par de la N-éthylmorpholine. 8,4 g (55 mM) de HOBT hydraté, puis 21 g de Boc–Gln–ONp (57 mM) sont ajoutés et le pH réajusté à 7. La température est ramenée à 20 °C et la réaction est maintenue à cette température 4 h, sous agitation et à un pH de 7 maintenu par de la N-éthyl-morpholine. Le solvant est évaporé sous vide, le résidu repris dans $H_2O$, essoré, lavé au tampon sulfate-bisulfate, lavé plusieurs fois à l'eau et séché à l'air. Le solide est broyé et lavé à l'acétate d'éthyle, essoré, relavé à l'eau et séché sous vide en présence de $P_2O_5$.

Rendement: 80% (39 g)

CCM: chloroforme/acétone/AcOH 75/18/7

7. Boc–Leu–Gln–Asp(OBz1)–Ile–Met–NH–NH–Troc

39 g (44 mM) Boc–Gln–Asp(OBz1)–Ile–Met–NH–NH–Troc ci-dessus sont introduits dans un mélange bien agité de 400 ml TFA glacé et 40 ml d'éthane-dithiol puis agités 45 min à TA. Le solvant est évaporé, le résidu est repris dans l'isopropanol et évaporé en terminant à la pompe à palettes. Par addition d'éther anhydre, le sel de TFA cristallise; il est essoré, lavé à l'éther et séché au dessiccateur en présence de potasse. Le sel de TFA ci-dessus est dissous dans 250 ml de DMF, le pH est ajusté à 7 (papier pH) par de la N-éthylmorpholine, 15 g (45,7 mM) de Boc–Leu–ONSu sont ajoutés et le pH est maintenu à 7 par de la N-éthyl-morpholine. Après 3 h 30, le DMF est évaporé sous vide et le résidu est repris dans l'eau. Le pH est amené à 2 par HCl 6 N. Le solide est homogénéisé, essoré, lavé à l'eau, séché, lavé 3 fois à l'acétate d'éthyle.

Rendement: 77% (34 g)

$^1$H-RMN: conforme

CCM: chloroforme-acétone-AcOH 75/18/7 tache unique

8. Boc–Leu–Gln–Asp(OBz1)–Ile–Met–NH–$NH_2$

17,9 g (17,5 mM) Boc–Leu–Gln–Asp(OBz1)–Ile–Met–NH–NH–Troc sont dissous dans un mélange de 200 ml de DMF et 50 ml d'acide acétique et agités énergiquement avec un agitateur mécanique. 11,5 g de zinc en poudre (300 mesh = 47 μm) sont ajoutés en une fois et l'agitation est poursuivie 1 h 40 à TA. Le solide est essoré, lavé au DMF et le filtrat est introduit dans 750 g de glace. L'ensemble est laissé au repos une nuit, puis essoré. Le précipité est lavé abondamment à l'eau et séché à l'air, puis au dessiccateur + P205.

Rendement: 78% (11,3 g)

$^1$H-RMN: conforme

Le produit obtenu est suffisamment pur(80–90%) pour l'étape suivante. Dans le cas contraire, il est purifié par dissolution dans le DMF et précipité par un égal volume de n-butanol.

Exemple VI

Boc–Arg(MTS)–Lys(Z)–Leu–NH–$NH_2$

1. Boc–Lys(Z)–Leu–$OCH_3$

A 1600 ml d'acétate d'éthyle refroidis à +10 °C par un bain extérieur de glace et sous agitation, on ajoute successivement: 53,88 g (0,297 mole) de H–Leu–$OCH_3$, HCl, 126 ml de N-éthyl-morpholine (1 mole), 44,5 g d'OHBT (0,33 mole) et finalement 166 g de Boc–Lys(Z)–OTcp (0,297mole). Le pH est maintenu à 6–7 par addition supplémentaire de N-éthyl-morpholine. Le milieu est agité 1 h à +10 °C, puis une nuit à la température ambiante. La réaction est alors terminée (CCM) et un léger insoluble est éliminé par filtration. La solution est lavée successivement avec 300 ml d'une solution aqueuse à 5% de $K_2SO_4$–$KHSO_4$, avec 300 ml d'une solution saturée de $NaHCO_3$ et finalement avec 300 ml d'une solution aqueuse de NaCl. La solution d'acétate d'éthyle est séchée sur $MgSO_4$ anhydre et le solvant évaporé sous vide à 30 °C. On obtient une huile qui est purifiée par chromatographie sur 4 kg de silice Fine Merck (230–400 mesh, soit 37–62 μm) dans une colonne de 7 × 210 cm. On élue d'abord avec le $CHCl_3$ pur jusqu'à élimination d'une impureté de tête. Le produit est élué par le mélange $CHCl_3$ à 2% de $CH_3OH$. Les fractions contenant le produit pur sont réunies et le solvant évaporé sous vide. Le résidu huileux est cristallisé dans le mélange pentane-éther 50–50. Le solide est essoré et séché. Le rendement en produit pur est de 119 g (79%).

$^1$H RMN: conforme.

2. Lys(Z)–Leu–O–$CH_3$, TFA

10,15 g (20 mM) de Boc–Lys(Z)–Leu–O–$CH_3$ sont mis en suspension dans 50 ml de chlorure de méthylène. Le mélange est refroidi à +5 – +10 °C et on ajoute 50 ml de TFA. On obtient ainsi une solution homogène qui est conservée 50 min à la température ambiante sous agitation. Le milieu est évaporé à sec sous vide sans chauffer (30 °C) et l'huile résiduelle lavée à l'éther et ce dernier évaporé à son tour.

Rendement: 18,1 g en un produit huileux utilisé tel quel dans l'étape suivante.

3. Boc–Arg(MTS)–Lys(Z)–Leu–$OCH_3$

9,14 g (0,02 mole) de Boc–Arg(MTS)–OH sont dissous dans 150 ml de THF. On refroidit au bain de glace et on ajoute 2,82 g de OHNSu (N-hydroxy succinimide) et 5,02 g de DCCl. Après 1 h, le précipité formé de dicyclohexylurée est essoré et on ajoute alors la solution dans 150 ml de

THF du produit obtenu dans l'exemple précédent (Exemple no. VI-2.). Le pH du milieu est maintenu à 7 par addition de NEM. Le milieu est maintenu sous agitation à la température ambiante pendant une nuit. La réaction est terminée (CCM) et le solvant évaporé sous vide à 30 °C. Le résidu est repris dans 200 ml d'acétate d'éthyle et lavé successivement avec 100 ml d'une solution aqueuse à 5% de $K_2SO_4$–$HKSO_4$, 100 ml d'une solution aqueuse saturée en $HNaCO_3$, 100 ml d'une solution aqueuse saturée en NaCl et finalement la phase organique est séchée sur $MgSO_4$ anhydre et le solvant évaporé sous vide. Le résidu huileux obtenu est purifié par chromatographie sur 600 g de silice Merck Fine (230–400 mesh, soit 37–62 µm) dans une colonne de 4 × 95 cm. On élue d'abord les impuretés avec le $CHCl_3$. Les fractions contenant le produit pur sont réunies et évaporées. Le produit s'organise en solide dans le pentane.

Rendement: 9 g (53%)

$^1H$ RMN conforme – CCM: ($CHCl_3$90, $CH_3OH$ 10) – Rf: 0,5

4. Boc–Arg(MTS)–Lys(Z)–Leu–NH–$NH_2$

7,1 g (0,0084 mole) de Boc–Arg(MTS)–Lys(Z)–Leu–$OCH_3$ sont dissous dans 110 ml de méthanol. Sous agitation magnétique et à la température ambiante, on ajoute 20 ml d'hydrate d'hydrazine. La réaction est complète en 3 h et le milieu réactionnel est versé sur de la glace pilée sous agitation. On laisse au repos une nuit et le solide est essoré et séché dans un dessiccateur sous vide en présence de $P_2O_5$. On obtient dans ces conditions 6,1 g (86%) d'un solide blanc.

$^1H$ RMN: conforme – CCM: $CHCl_3$–$CH_3OH$ 90–10 – Rf: 0,25 –

Exemple VII

Boc–Gln–Leu–Ser–Ala–OMe (fragment $F_1$)

1. Boc–Ser–Ala–OMe

Dissoudre 25 g de H–Ala–OMe, HCl et 66 g de Boc–Ser–ONb dans 500 ml de DMF. Refroidir au bain de glace et ajouter sous agitation magnétique 25 ml de NEM, 24 g de N-hydroxybenzotriazole et maintenir le pH à 6,5 ou 7 par addition de NEM. Agiter 18 h à TA. Après contrôle CCM, évaporer le milieu à 90% (13,33 Pa – 35 °C). L'huile obtenue est dissoute dans 1500 ml de chloroforme. Laver avec une solution de chlorure de sodium saturée (2 fois), solution sulfate/bisulfate de potassium 5% 3 fois, bicarbonate de sodium saturée 5 fois. La solution est séchée sur $Na_2SO_4$ et évaporée à sec. On obtient une huile qui est séchée à poids constant (30 °C–13,33 Pa).

Rendement: 53,78 g – Contrôle CCM –

2. H–Ser–Ala–OMe, TFA

Dissoudre 52,8 g de Boc–Ser–Ala–OMe dans 100 ml de chlorure de méthylène sous agitation magnétique en refroidissant au bain de glace. Ajouter 250 ml de TFA refroidi et agiter 20 min à TA. Filtrer. Evaporer le filtrat à sec (35° trompe à eau). Par addition de 2 l d'éther, un solide blanc précipite. Il est essoré, lavé à l'éther 3 fois. Sécher à poids constant (30 °C – 13,33 Pa).

Rendement: 40,82 g

3. Boc–Leu–Ser–Ala–OMe

Dissoudre 40 g de H–Ser–Ala–OMe, TFA et 32,9 g de Boc–Leu–OH, $H_2O$ dans 400 ml de DMF. Refroidir au bain de glace sous agitation magnétique et ajouter 14,4 ml de NEM puis 65,8 g de BOP et assez de NEM pour maintenir le pH à 6,5–7. Agiter 2 h à TA. Après contrôle CCM, évaporer à sec (13,33 Pa – 35 °C) et dissoudre l'huile obtenue dans 1500 ml de chloroforme. Laver 2 fois avec une solution NaCl saturée, 3 fois avec sulfate/bisulfate de potassium 5%, 3 fois au bicarbonate de sodium saturé. Sécher/$Na_2SO_4$. Evaporer à sec (30 °C–trompe à eau). L'huile obtenue est solidifiée dans l'éther: Fraction A = 29 g. Un deuxième jet B est obtenu par addition d'hexane, identique en CCM: B = 13,7 g.

Rendement: 42,7 g – Contrôles RMN et CCM.

4. H–Leu–Ser–Ala–OMe, TFA

Mettre en suspension 29 g de Boc–Leu–Ser–Ala–OMe dans 50 ml de chlorure de méthylène sous agitation magnétique en refroidissant au bain de glace. Ajouter 100 ml de TFA refroidi et, après dissolution, agiter 30 min à TA. Filtrer. Evaporer le filtrat à sec (35 °C – trompe à eau). L'huile obtenue est solidifiée dans l'éther (changer le solvant plusieurs fois). Sécher à poids constant (30 °C – 13,33 Pa).

Rendement: 30,2 g.

5. Boc–Gln–Leu–Ser–Ala–OMe

Dissoudre sous agitation magnétique 30 g de H–Leu–Ser–Ala–OMe, TFA et 25,5 g de Boc–Gln–ONp dans 300 ml de DMF. Neutraliser par 9,7 ml de NEM. Ajouter 10 g d'OHBt et agiter 2 h à TA en maintenant le pH à 6,5–7 par addition de NEM. Après contrôle CCM, le milieu est concentré à 90% (13,33 Pa – 35 °C), repris dans 1000 ml de chloroforme: un produit précipité en gel. Essorer. Laver en reprenant dans 500 ml de chloroforme 2 fois, puis dans l'éther 3 fois. Sécher à poids constant (13,33 Pa – 30 °C).

Rendement: 32,1 g

CCM: chloroforme/MeOH/AcOH: 9/2/0,5 – Rf = 0,81 –

HPLC – EPP = 91%

AAA: Ser: 0,86 – Glu: 1,02 – Ala: 0,99 – Leu: 0,99 –

Exemple VIII

Boc–Tyr–Arg–Lys(Z)–Val–Leu–Gly–OH, HCl (fragment $G_1$)

1. Z–Leu–Gly–$OCH_3$

On dissout 0,22 mole de Z–Leu–OH dans 500 ml de DMF. A cette solution, on ajoute successivement:

- 25,11 g de HCl–H–Gly–$OCH_3$ (0,2 mole)
- 28 ml de NEM
- 96 g de Bop (0,22 mole)

puis on ajuste le pH à 7 par addition de NEM.

On agite le mélange réactionnel à TA en maintenant le pH à 7 par addition de NEM si nécessaire. La réaction est suivie en CCM (chloroforme-méthanol-acide acétique 95/5/9). Au bout de 2 h, la réaction est terminée. Le mélange réactionnel est évaporé à sec sous pression réduite (26,66 Pa) et à température inférieure à 30 °C. Le résidu est repris dans l'acétate d'éthyle (1 litre). La solution est lavée successivement avec:

– solution aqueuse de bicarbonate (4 × 200 ml)

– solution aqueuse de $SO_4HK$–$SO_4K_2$ (4 × 200 ml)

– solution aqueuse saturée de chlorure de sodium (2 × 200 ml)

puis, elle est séchée sur sulfate de magnésium et évaporée à sec sous pression réduite. Le résidu est repris dans de l'éther (500 ml), trituré, essoré, séché.

Rendement: 56,7 g (84,28%)

CCM chloroforme-méthanol-acide acétique 95/5/3 – Rf: 0,44

2. HCl–H–Leu–Gly–$OCH_3$

On dissout 56 g (0,166 mole) de Z–Leu–Gly–$OCH_3$ dans 900 ml de méthanol chlorhydrique 0,23 N. A cette solution, on ajoute 6 g de Pd/C à 10% contenant 50% d'humidité. On hydrogène pendant 24 h, à TA et sous pression atmosphérique. On filtre le catalyseur et la solution est évaporée à sec sous pression résuite à température inférieure à 30 °C. On reprend le résidu par de l'éther (2 × 50 ml), on décante et on séche le produit au dessiccateur en présence d'anhydride phosphorique. On obtient une poudre blanche.

Rendement: 33,53 g (84,4%)

CCM chloroforme-méthanol-acide acétique 90/20/9 – Rf: 0,15 – Contrôle RMN

3. Boc–Val–Leu–Gly–$OCH_3$

28,24 g de Boc–Val–OH (0,13 mole) sont dissous dans 400 ml de tétrahydrofuranne refroidi à −10 °C. On ajoute ensuite 14,45 ml (0,13 mole) de chloroformiate d'isobutyle. Le mélange est vigoureusement agité à −10 °C pendant 15 min. A ce mélange, on ajoute une solution tétrahydrofurannique refroidie à −10 °C contenant 31,5 g du chlorhydrate de H–Leu–Gly–$OCH_3$ (0,13 mole) prélablement neutralisé par 14,45 ml de N-méthylmorpholine. On poursuit l'agitation en refroidissant le mélange réactionnel dans un bain de glace (2 h) puis à TA pendant 2 h. Le mélange réactionnel est évaporé à sec sous pression réduite à température inférieure à 30 °C. Le résidu est repris dans un mélange acétate d'éthyle-eau (500 ml – 500 ml). La phase aqueuse est décantée et la phase organique lavée successivement avec:

– une solution aqueuse de bicarbonate de soude (2 × 50 ml)

– une solution aqueuse de $SO_4HK$–$SO_4K_2$ (pH 2) (2 × 50 ml)

– une solution aqueuse de chlorure de sodium (50 ml), puis séchée sur sulfate de magnésium et évaporée à sec sous pression réduite à température inférieure à 30 °C. Le résidu est repris avec le pentane (50 ml), essoré et séché sous vide (13,33 Pa).

Rendement: 39,7 g (76%)

CCM chloroforme-acétone 75/25 – Rf: 0,46

Contrôle RMN

4. TFA H–Val–Leu–Gly–$OCH_3$

On dissout 39 g de Boc–Val–Leu–Gly–$OCH_3$ (0,097 mole) dans 190 ml de TFA refroidi dans un bain de glace. Après dissolution, on enlève le bain de glace et on agite le mélange pendant 30 min à TA. On évapore à sec sous pression réduite et le résidu est repris dans l'éther (100 ml), trituré et l'éther décante. Cette opération est répétée 2 fois et les dernières traces de solvants sont éliminées sous pression réduite (13,33 Pa).

Rendement: 44,8 g

CCM chloroforme-méthanol-acide acétique 90/20/3 – Rf: 0,36.

5. Boc–Lys(Z)–Val–Leu–Gly–$OCH_3$

On dissout 25,55 g de TFA H–Val–Leu–Gly–$OCH_3$ (0,0615 mole) dans 200 ml de DMF. La solution est amenée à pH 7 par addition de NEM. On ajoute alors à cette solution 32,47 g de Boc–Lys(Z)–OTCP (0,058 mole) et 9 g de HOBT (0,059 mole). Le pH du mélange réactionnel est ramené à 7 par addition de NEM. On agite à TA en maintenant le pH à 7 par addition de NEM si nécessaire. La réaction est suivie en CCM: chloroforme-méthanol 90/10; chloroforme-méthanol-acide acétique 90/20/3 (2 milieux).

Au bout de 1 heure, on rajoute 1,96 g de Boc–Lys(Z) OTCP (0,0035 mole) et 0,61 g de HOBT. Le pH de la solution est ramené à 7 par addition de NEM.

2 heures après, la réaction est terminée en CCM. Le mélange réactionnel est alors évaporé à sec sous pression réduite (0,1 mm de mercure) à température inférieure à 30 °C. Le résidu est repris avec 500 ml d'eau, trituré. Le solide formé est essoré puis lavé avec:

– 500 ml de solution aqueuse de $HKSO_4$–$K_2SO_4$ (pH 2)

– 500 ml d'une solution aqueuse saturée de bicarbonate de soude

– 200 ml d'eau

puis à l'éther (200 ml × 2) et séché.

Le produit impur est chromatographié sur une colonne de gel de silice (5 cm × 150 cm). Il est élué avec le mélange chloroformeacétate d'éthyle 80/20 (débit 600 ml/h). Les bonnes fractions déterminées en CCM sont réunies, évaporées à sec sous pression réduite. Le résidu est repris dans l'éther, trituré et le solide obtenu essoré. Poudre blanche 35,14 g (86%)

CCM chloroforme-méthanol 90/10 – Rf: 0,62

Chloroforme-méthanol-acide acétique 87,7–9,4–2,8 Rf: 0,52

Contrôle RMN

AAA: Gly 1,00–Val 0,96–Leu 1,06–Lys 0,98

6. TFA H–Lys(Z)–Val–Leu–Gly–$OCH_3$

On dissout 15 g de Boc–Lys(Z)–Val–Leu–Gly–$OCH_3$ (22,6 mM) dans un mélange de TFA-chlorure de méthylène (75 ml/75 ml). On agite 40 min à TA, puis on évapore à sec sous pression réduite à température inférieure à 30 °C. Le résidu est repris par de l'éther contenant 20% d'hexane (100 ml). Le solide formé est essoré et séché. On obtient un solide blanc de 14,51 g.

Rendement: 94,7%

CCM chloroforme/méthanol/acide acétique 95/5/3 – Rf: 0,12 –

7. Boc–Arg(HCl)–Lys(Z)–Val–Leu–Gly–$OCH_3$

On dissout 14,1 g de TFA H–Lys(Z)–Val–Leu–Gly–$OCH_3$ (21 mM) dans 50 ml de DMF. La solution est amenée à pH par addition de NEM. On ajoute alors à cette solution 6,57 g de Boc–Arg (HCl)–OH.1 $H_2O$ (20 mM) et 10,6 g de BOP (24 mM). Le pH du mélange réactionnel est ramené à 6–7 par addition de NEM. On agite à TA en maintenant le pH à 6–7 par addition de NEM si nécessaire. La réaction est suivie en CCM: chloroforme/méthanol (80/20).

Au bout de 5 h, la réaction est terminée. On évapore à sec le mélange réactionnel sous pression réduite (13,33 Pa) et à température inférieure à 30 °C. Lhuile obtenue est reprise dans 300 ml d'acétate d'éthyle. Le solide formé est essoré et lévé à l'éther (1er jet). On rajoute de l'éther au filtrat précédent; un solide précipite qui est essoré (2ème jet): les 2 jets sont identiques en CCM. Ils sont lavés à l'état solide par agitation pendant 1 h avec un mélange de 200 ml d'eau saturée en acétate d'éthyle et 60 ml d'eau. Le solide est essoré puis lavé avec 60 ml d'eau et 300 ml d'éther et séché.

Rendement: 78,7% (13,49 g)

CCM chloroforme/méthanol 20/20 – Rf: 0,32 – Contrôle RMN

8. TFA H–Arg(HCl)–Lys(Z)–Val–Leu–Gly–$OCH_3$

13,3 g de Boc–Arg(HCl)–Lys(Z)–Val–Leu–Gly–$OCH_3$ (15,5 mM) sont dissous dans un mélange TFA-chlorure de méthylène (60 ml/60 ml). On agite 40 min à TA, puis on évapore à sec sous pression réduite à température inférieure à 30 °C. Le résidu est repris par un mélange éther-hexane (80/20: 100 ml). Le solide formé est essoré, lavé à l'hexane et séché (poids: 15,13 g).

CCM: chloroforme/méthanol (3/1) – Rf: 0,35 –

9. Boc–Tyr–Arg(HCl)–Lys(Z)– Val–Leu–Gly –$OCH_3$

On dissout 15,5 mM de TFA H–Arg(HCl)–Lys(Z)–Val–Leu–Gly–$OCH_3$ dans 50 ml de DMF. La solution est amenée à pH 6 par addition de NEM. On ajoute alors à cette solution 8,20 g de Boc–Tyr–OTCP (15,5 × 10 mM) et 2,41 g de HOBT (15,5 × 10 mM). On ramène le pH à 6 par addition de NEM. Le mélange réactionnel est agité à TA en maintenant le pH à 6 par addition de NEM si nécessaire. La réaction est suivie en CCM: chloroforme/méthanol (3/1). Au bout de 3 h, la réaction est terminée. On évapore à sec le mélange réactionnel, sous pression réduite (13,33 Pa)et à température inférieure à 30 °C. L'huile résiduelle est reprise dans de l'acétate d'éthyle (300 ml). Le solide gélatineux formé est essoré, lavé avec de l'acétate d'éthyle (100 ml), avec de l'acétate d'éthyle-éther (1/1; 100 ml), puis à l'éther (100 ml), enfin à l'hexane (100 ml). Le produit est séché sous pression réduite jusqu'à poids constant.

Rendement: 96% (15,14 g).

CCM chloroforme/méthanol (3/1) – Rf: 0,47 – Contrôle RMN

AAA: Gly: 0,95 – Val: 0,98 – Leu: 1,02 – Lys: 1,01 –

Arg: 1,01 – Tyr: 1,05 –

HPLC: 93,81% EPP.

10. Boc–Tyr–Arg(HCl)–Lys(Z)–Val–Leu–Gly–OH

7 g de Boc–Tyr–Art(HCl)–Lys(Z)–Val–Leu–Gly–$OCH_3$ (6,86 mM) sont dissous dans un mélange de 70 ml de dioxane et 35 ml d'eau. On ajoute à cette solution 6,4 ml de soude 4N (25,6 mM): 3,7 équivalents et on agite pendant 30 min à TA. On dilue avec 200 ml d'eau et 800 ml d'acétate d'éthyle. Le mélange est alors acidifié à pH 3 par addition de HCl (N). On décante l'acétate d'éthyle et on essore le solide (1er jet). L'acétate d'éthyle est évaporé à sec; on obtient un 2ème jet. Les 2 jets identiques en CCM sont réunis, lavés à l'éther et séchés.

Rendement: 74,7% (5,16 g)

CCM: chloroforme/méthanol (2/1) – Rf: 0,45

1H Contrôle RMN

AAA: Gly: 1,00 – Val: 0,97 – Leu: 1,04 – Tyr: 0,95 –

Lys: 0,99 – Arg: 1,05 –

HPLC – EPP 91,52%.

Exemple IX

Boc–Ile–Phe–Thr–Asn–Ser–NH–$NH_2$(fragment $H_1$)

1. Boc–Asn–Ser–OMe

Une solution de 23,32 g de Boc–Asn–OH (0,1 M) et 19,7 g de NbOH (0,11 M) dans 250 ml de DMF, refroidie sur bain de glace est additionnée de 22,69 g de DCC. Après 3 h de réaction à TA, on filtre la DCU, on refroidit sur bain de glace et ajoute 15,56 g de HCl, H–Ser–OMe (chlorhydrate de H–Ser–OMe). Le pH est amené, puis maintenu à 7 par addition de NEM. Après 4 h à TA et 20 h à +4°, la solution est concentrée sous pression réduite. Le résidu est repris par le mélange AcOEt/n-butanol (50/50). La solution organique est lavée successivement par:

– une solution saturée de bicarbonate de sodium
– une solution saturée de chlorure de sodium
– une solution de $KHSO_4/K_2SO_4$ à 5%
– une solution saturée de chlorure de sodium.

Après séchage sur $MgSO_4$ et évaporation sous pression réduite, le résidu est cristallisé dans le mélange éther-hexane. Filtration, lavage à éther/hexane et séchage sous vide donnent 26,9 g de produit.

Rendement: 80,8%

Contrôles RMN et CCM

2. TFA, H–Asn–Ser–OMe

Une solution refroidie sur bain de glace de 13,5 g de Boc–Asn–Ser–OMe (40,5 mM) dans 54 ml de dichlorométhane est additionnée de 81 ml de TFA. Après 1 h de réaction à TA, la solution est concentrée sous pression réduite, le résidu est repris à l'éther. On obtient une huile épaisse qui est utilisée dans la réaction suivante.

3. Boc–Thr–Asn–Ser–OMe

Une solution refroidie sur bain de glace du produit brut précédemment obtenu dans 300 ml de DMF est additionnée de 14,09 g de Boc–Thr–ONSu (44,55 mM) 5,58 g d'HOBt (41,3 mM) et de NEM jusqu'à obtenir un pH de 7. Après 20 h de réaction à TA, la solution est concentrée sous pression réduite et le résidu est repris dans le mélange AcOEt/n butanol. La solution organique est lavée successivement par:

– une solution saturée de bicarbonate de sodium
– une solution saturée de chlorure de sodium
– une solution de $KHSO_4/K_2SO_4$ à 5%
– une solution saturée de chlorure de sodium.

Après séchage sur $MgSO_4$, la solution est concentrée sous pression réduite et le résidu cristallisé dans le mélange éther/hexane. On obtient 8,28 g de produit.

Rendement: 47%

Contrôles RMN et CCM

4. TFA, H–Thr–Asn–Ser–OMe

Une solution refroidie sur bain de glace 8 g de Boc–Thr–Asn–Ser–OMe dans 32 ml de dichlorométhane est additionné de 54 ml de TFA. Après 1 h de réaction à TA, la solution est concentrée sous pression réduite et le résidu précipité à l'éther. Après filtration, il est utilisé tel quel dans la réaction suivante.

5. Boc–Phe–Thr–Asn–Ser–OMe

Une solution du précipité obtenu à l'étape précédente dans 100 ml de DMF est additionnée sous refroidissement par bain de glace de 7,82 g de Boc–Phe–ONp (20,24 mM), 2,53 g d'HOBt (18,71 mM) et de la N-éthylmorpholine jusqu'à obtenir un pH de 7. Après 4 h, la solution est concentrée sous pression réduite et le résidu est repris dans AcOEt. La solution organique est lavée par:

–une solution saturée de bicarbonate de sodium
– une solution saturée de chlorure de sodium
– une solution de $KHSO_4/K_2SO_4$ à 5%
– une solution saturée de chlorure de sodium.

Après séchage sur $MgSO_4$, concentration de la phase organique le résidu est cristallisé dans l'éther. On obtient 10 g de produit.

Rendement: 93,5%

Contrôles CCM et RMN.

6. TFA, H–Phe–Thr–Asn–Ser–OMe

Une solution de 10 g de Boc–Phe–Thr–Asn–Ser–OMe (17,19 mM) dans 40 ml de dichlorométhane est additionnée sous refroidissement par bain de glace de 60 ml de TFA. Après 1 h de réaction à TA, le produit est précipité à l'éther. La gomme obtenue après séchage précipite en donnant 5,14 g de produit.

Rendement: 48,5%

7. Boc–Ile–Phe–Thr–Asn–Ser–OMe

Une solution refroidie sur bain de glace de 5,14 g de TFA, H–Phe–Thr–Asn–Ser–OMe (8,78 mM) dans 100 ml de DMF est additionnée de 3,17 g de Boc–Ile–ONSu(9,66 mM), 1,30 g d'HOBt (9,62 mM) et de N-éthylmorpholine jusqu-à obtenir un pH de 7. Après 20 h de réaction à TA, la solution est concentrée sous pression réduite et le résidu repris à l'éther donne un précipité qui est filtré et séché sous vide. On obtient 5,24 g de produit.

Rendement: 85,9%

Contrôle RMN et HPLC.

8. Boc–Ile–Phe–Thr–Asn–Ser–NH–$NH_2$

Une solution de 5 g de Boc–Ile–Phe–Thr–Asn–Ser–OMe (7,2 mM) dans 40 ml de DMF et 200 ml de méthanol refroidie sur bain de glace est additionnée de 3,6 ml d'une solution à 80% d'hydrate d'hydrazine. Après 21 h de réaction à TA, on ajoute 0,9 ml de la même solution d'hydrate d'hydrazine. Après 3 h supplémentaires, le gel obtenu est filtré, lavé au méthanol et séché sous vide. On obtient 3,94 g de produit.

Rendement: 78,8%

Contrôles RMN et CCM.

Exemple X

Boc–Tyr–Ala–Asp(OBz1)–Ala–OH (fragment I)

1. Boc–Asp(OBz1)–Ala–OBut

3,62 g (0,02 mole) de chlorhydrate d'alaninate de tertiobutyle sont mis en suspension dans 30 ml d'acétonitrile. 2,52 ml de NEM sont ajoutés puis 8,4 g (0,02 mole) de Boc–Asp(OBz1) ONSu. Le pH est maintenu à 6–6,5 par addition de NEM. L'agitation est poursuivie 18 h. Après contrôle CCM le milieu est évaporé à sec, l'huile est reprise dans 50 ml AcOEt, la solution lavée 2 fois par une solution $KHSO_4/K_2SO_4$, puis à l'eau salée, séchée sur $Na_2SO_4$ et évaporée à sec. L'huile obtenue est dissoute dans l'hexane, un peu de solide est filtré et le filtrat est évaporé à sec. L'huile obtenue est utilisée telle quelle.

CCM: $CHCl_3$/Acétone/ACOH 80/15/5 – Rf: 0,8–

2. Boc–Ala–Asp(OBz1)–Ala–OH

L'huile brute obtenue précédemment est dissoute dans 50 ml TFA glacé et agitée 1 h 30 à TA. Le TFA est évaporé au maximum et l'huile résiduelle est reprise 3 fois dans l'éther anhydre et décantée. Elle est ensuite séchée au dessiccateur sous vide en présence de pentane. Il se forme une mousse solide qui est écrasée et remise à sécher.

On obtient 9,4 g de produit brut utilisé tel quel pour le couplage.

Le sel de TFA ci-dessus est dissous dans un mélange de 20 ml DMF et 18 ml d'eau distillée. Le NEM est ajoutée jusqu'à pH 7 (utilisation du

pHmètre). 4 g (0,014 mole) de Boc–Ala–ONSu en solution dans 16 ml de DMF sont introduits. Le pH est maintenu à 6,5–7 par addition de NEM. Après 5 h 30, la réaction est terminée (contrôle CCM). Le solvant est évaporé au maximum et le résidu est repris dans un mélange d'eau et d'acétate d'éthyle. La phase organique est lavée par une solution de $NaHCO_3$, à l'eau salée, séchée sur $NA_2SO_4$ et évaporée à sec. Le résidu est dissous dans l'éther et précipité par l'hexane pour donner une gomme qui cristallise après triturage. Le solide est essoré et séché à l'air.

Rendement: 5,05 g.

CCM $CHCl_3$-acétone-ACOH 80/15/5 – Rf: 0,28

3. Z–Tyr–Ala–Asp(OBz1)–Ala–OH

1 g (2 mmoles) de Boc–Ala–Asp(OBz1)–Ala–OH est introduit dans 10 ml TFA glacé agité. Après 30 min d'agitation à TA, le TFA est évaporé au maximum, le résidu repris 2 fois dans l'éther anhydre et décanté, puis séché au dessiccateur sous vide en présence de potasse. Il se forme une mousse solide. Le produit brut est mis en solution dans un mélange de 4 ml DMF et 2 ml d'eau distillée. NEM est ajouté jusqu'à pH 7–7,5; 1 g (2,4 mmoles) de Z–Tyr–ONSu est alors ajouté et le pH est maintenu à 7–7,5 par NEM (pHmètre). Au bout de 2 h 30, après contrôle CCM, le solvant est évaporé au maximum á 40 °C. Le résidu est repris dans l'acétate d'éthyle et lavé à l'eau, un peu de solide est éliminé et l'acétate d'éthyle est lavé au mélange sulfate/bisulfate et à l'eau salée. Après abandon quelques heures, il se forme un précipité qui est essoré, lavé à l'éther et séché à l'air. 500 mg sont ainsi obtenus. Un 2ème jet de 350 mg légèrement moins pur est obtenu par concentration de l'acétate d'éthyle.

CCM chloroforme-ACOH 3/1 RF: 0,4
butanol-ACOH-$H_2O$ 72/7/2 Rf: 0,85
chloroforme-acétone-ACOH 80/15/5 Rf: 0,1
HPLC 90,9% pureté polypeptidique
Contrôle RMN

Exemple XI

Synthèse de H–Arg(MTS)–Lys(Z)–Leu–Leu–Gln–Asp(OBz1)–Ile–Met–Ser–Arg–Gln–Gly–Glu(OBz1)–Ser–Asn–Gln–Slu(OBz1)–Arg–Gly–Ala–Arg–Ala–Arg–Leu–$NH_2$ TFA, HCl (Peptide $K_1$

1. Boc–Gln–Glu(OBz1)Arg–Gly–Ala–Arg–Ala–Arg–Leu–$NH_2$, HCl

6,53 g de H–Ala–Arg–Ala–Arg–Leu–$NH_2$, HCl (fragment A), soit $10^{-2}$ mole et 7,5 g de Boc–Gln–Glu(OBz1)–Arg–Gly–OH (1,05 × $10^{-2}$ mole) sont dissous dans 100 ml de DMF. Le pH est ramené à 7 par addition de NEM et ensuite on ajoute 5,2 g de BOP ($10^{-2}$ mole + 20%) sous agitation et à température ambiante. Le pH est maintenu à 7 par additions intermittentes de NEM. La réaction est terminée au bout de 4 h. Le milieu est concentré sous vide à 35 °C et, au résidu d'évaporation, on ajoute de l'acétate d'éthyle. Un solide précipite. Il est essoré, lavé à l'acétate d'éthyle (3 fois), lavé à l'acétonitrile (3 fois), à l'éther et séché sous vide jusqu'à poids constant. On obtient 11,6 g (87%) d'un produit blanc.

CCM: Butanol-pyridine-AcOH-eau (50/12/12/25) – Rf: 0,81

AcOEt-pyridine-$HCO_2H$-eau (40/20/10/6) – Rf: 0,89

AAA: Glu (Gln): 2,02 – Arg: 3,02 – Leu: 0,95 – Gly: 0,91 – Ala: 2,1 –

2. H–Gln–Glu(OBz1)–Arg–Gly–Ala–Arg–Ala–Arg–Leu–$NH_2$, TFA, HCl

11 g du précédent peptide (Exemple XI no. 1) sont mis en suspension dans 50 ml de chlorure de méthylène froid. On ajoute sous agitation magnétique 60 ml de TFA préalablement refroidis au bain de glace. Après dissolution, le milieu est agité 30 min. à la température ambiante. On ajoute ensuite 500 ml d'éther, et un solide pulvérulent blanc précipite instantanément. Il est essoré, lavé 4 fois avec 200 ml d'éther et séché jusqu'à poids constant. On obtient dans ces conditions un rendement quantitatif (11 g) d'un produit blanc.

CCM: Butanol-pyridine-AcOH-eau (50/12/12/25) – Rf: 0,29

AcOEt-Pyridine-$HCO_2H$-eau (63/21/10/6) – Rf: 0,11

3. Boc–Glu(OBz1)–Ser–Asn–Gln–Glu(OBz1)–Arg–Gly–Ala–Arg–Ala–Arg–Leu–$NH_2$, HCl

On dissout sous agitation magnétique 10,9 g (8,1 × $10^{-3}$ mole) du précédent produit (Exemple XI, 2°) dans 120 ml de DMF. Le pH est amené à 7 par addition de NEM. On ajoute 6,49 g de Boc–Glu(OBz1)–Ser–Asn–OH et 4,35 g de BOP (20% d'excès) sous agitation et à la température ambiante. Le pH est maintenu à 7 par addition de NEM. La réaction est terminée au bout de 3 h. On concentre sous vide à 35 °C et le résidu est précipité par l'acétate d'éthyle. Le solide formé est essoré et lavé 3 fois à l'acétate d'éthyle (3 × 100 ml), 3 fois à l'acétonitrile (3 × 100 ml), à l'éther et séché jusqu'à poids constant. On obtient 12,6 g (90%) d'un solide blanc. Contrôles HPLC

CCM: butanol – pyridine-AcOH-eau (60/12/12/25) – Rf: 0,49 –

AcOEt-pyridine-$HCO_2H$-eau (63/21/10/60) – Rf: 0,40 –

AAA: Glu(Gln): 3,2 – Ser: 0,92 – Arg: 2,93 – Leu: 0,98 –

Asp(Asn): 0,98 – Gly: 0,96 – Ala: 2,00 –

4. H–Glu(OBz1)–Ser–Asn–Gln–Glu(OBz1)–Arg–Gly–Ala–Arg–Ala–Arg–Leu–$NH_2$, TFA, HCl

On met en suspension sous agitation magnétique, dans 50 ml de chlorure de méthylène refroidis extérieurement par un bain de glace, 12,4 g du précédent peptide (Exemple XI, 3°). On introduit 50 ml de TFA préalablement refroidi à 0 °C. Le produit se dissout et on maintient l'agitation 30 min à T.A. On ajoute alors 500 ml d'éther. Un solide blanc précipite. Il est essoré, lavé quatre fois avec 100 ml d'éther et séché sous vide jusqu'à poids constant. On obtient ainsi 12,5 g (Rendement quantitatif) d'un produit blanc.

CCM: butanol-pyridine-AcOH-eau (4/2/1/2) – Rf: 0,08 –

5. Boc–Ser–Arg–Gln–Gln–Gly–Glu(OBz1)–Ser–Asn–Gln–Glu(OBz1)–Arg–Gly–Ala–Arg–Ala–Arg–Leu–$NH_2$, HCl

On dissout 1,74 g ($10^{-3}$ mole) du précédent produit (Exemple XI, 4°) dans 100 ml de DMF. On amène le pH à 7 par de la NEM. On porte le milieu sous agitation magnétique à 35 °C et on ajoute 0,48 g de BOP et 0,783 g de Boc–Ser–Arg–Gln–Glu–Gly–OH($10^{-3}$ mole + 10%) par petites portions pendant 1/2 h. On rajoute ensuite 0,9 g de BOP en maintenant la température à 35 °C et le pH à 6,5 par addition de NEM. L'état d'avancement de la réaction est suivi par HPLC. La réaction est terminée en 1 h et le milieu est versé dans 200 ml d'acétate d'éthyle et laissé une nuit au repos. Un solide s'organise. Il est essoré, lavé à l'acétate d'éthyle (3 × 50 ml), à l'éther et séché jusqu'à poids constant. Le rendement est de 2 g en un produit pulvérulent blanc.

CCM: Butanol-pyridine-AcOH-eau (4/2/1/2) – Rf: 0,44

AAA: Ser: 1,91 – Arg: 3,88 – Glu(Gln): 4,82 – Gly: 2,01 – Asp(Asn): 0,91 – Ala: 2,00 – Leu: 0,98 –

6. H–Ser–Arg–Gln–Gln–Gly–Glu(OBz1)–Ser–Asn–Gln–Glu(OBz1)–Arg–Gly–Ala–Arg–Ala–Arg–Leu–$NH_2$, TFA, HCl

25 g du peptide décrit dans l'exemple XI, 5° sont mis en suspension dans 100 ml de chlorure de méthylène refroidis à 0 °C. On ajoute 50 ml de TFA à 0 °C et, après la fin de la dissolution, on conserve le milieu à la température ambiante 30 min. On ajoute alors 500 ml d'éther. Un solide blanc précipite. Il est essoré; lavé 4 fois avec 200 ml d'éther et séché sous vide jusqu'à poids constant. On obtient 25 g (rendement quantitatif) d'un solide pulvérulent blanc. CCM: Butanol-pyridine-AcOH-eau (4/2/1/2) – Rf: 0,05

7. Boc–Leu–Gln–Asp(OBz1)–Ile–Met–Ser–Arg–Gln–Gln–Gly–Glu(OBz1)–Ser–Asn–Gln–Glu(OBz1)–Arg–Gly–Ala–Arg–Ala–Arg–Leu–$NH_2$, HCl

a) Boc–Leu–Gln–Asp(OBz1)–Ile–Met–NH–$NH_2$

13,64 g de Boc–Leu–Gln–Asp(OBz1)–Ile–Met–NH–$NH_2$ (0,0166 mole) sont dissous dans 100 ml de DMSO anhydre et 200 ml de DMF anhydre. La solution est refroidie entre −15 et −20 °C sous agitation et sous atmosphère inerte (azote). On ajoute 8 ml d'une solution 8,25 N de HCl dans le dioxanne et immédiatement après 2,5 ml de nitrite de t.-butyle (soit 1,2 équivalent). La solution obtenue est agitée sous atmosphère inerte ($N_2$) 50 min entre −15 et −20 °C. Le pH du milieu est amené à 6,3 – 6,6 par addition de DIPEA. 1/4 de la solution obtenue est prélevé et conservé au congélateur à −25 °C. Le reste est engagé dans la phase suivante.

b) Couplage de l'azide

20,06 g de peptide obtenu dans l'exemple XI, 6° (8,28 × $10^{-3}$ mole) sont dissous dans 70 ml de DMSO, on ajoute ensuite 100 ml de DMF. Le milieu est refroidi à −10 °C et le pH est amené à 6,8 par addition de DIPEA. Cette solution refroidie est coulée ensuite en 30 min dans les 3/4 de la précédente solution d'azide obtenue dans l'exemple XI, 6° (a), en maintenant la température entre −20 et −15 °C. Le pH est ajusté à 7 par addition de DIPEA. Le milieu est conservé à −15 °C pendant 24 h. Après cet intervalle de temps, le pH qui était tombé à 6 est ramené à 7 par la DIPEA et on rajoute le reste de la solution d'azide (le 1/4 qui avait été conservé). Le pH est ramené à 7 par addition de DIPEA. Après 24 h de conservation supplémentaire à cette température (−20 −15 °C) le milieu est versé sur 2 l d'acétate déthyle refroidi et sous agitation. L'agitation est poursuivie 30 min et le solide formé essoré est lavé par 5 fois 300 ml d'acétate d'éthyle et 3 fois 300 ml d'éther et séché une nuit au dessiccateur sous vide jusqu'à poids constant. On obtient ainsi un produit pulvérulent blanc pesant 22,08 g (Rendement: 87,16%). CCM: $BPEW_1$ – Rf: 0,5

AAA: Leu: 1,98 – Glu(Gln): 5,92 – Asp(Asn): 1,98 – Ile: 0,92 – Met: 0,89 – Ser: 1,86 – Arg: 3,92 – Gly: 2,02 – Ala: 2,02 –

8. H–Leu–Gln–Asp(OBz1)–Ile–Met–Ser–Arg–Glu–Gln–Gly–Glu(OBz1)–Ser–Asn–Gln–Glu(OBz1)–Arg–Gly–Ala–Arg–Ala–Arg–Leu–$NH_2$, TFA, HCl

On met en suspension 10,25 g du peptide obtenu dans l'exemple précédent (XI, 7°) dans 80 ml de chlorure de méthyléne contenant 4 ml d'éthane dithiol. La suspension est agitée à température ambiante sous courant d'azote. On ajoute 80 ml de TFA. On obtient une solution bien homogène qui est conservée 40 min. Le milieu est concentré au 1/3 de son volume initial sous vide à 25 °C et coulé dans 400 ml d'éther refroidi à 0 °C sous agitation. On obtient un précipité qui est essoré et lavé 3 fois avec 100 ml d'éther et séché sous vide jusqu'à poids constant. On obtient 9,89 g(96,1%) d'un produit pulvérulent blanc.

CCM: $BPEW_1$ – Rf: 0,5

9. Boc–Arg(MTS)–Lys(Z)–Leu–Leu–Gln–Asp(OBz1)–Ile–Met–Ser–Arg–Gln–Gln–Gly–Glu(OBz1)–Ser–Asn–Gln–Glu(OBzl)–Arg–Gly–Ala–Arg–Ala–Arg–Leu, $NH_2$, HCl

a) Boc–Arg(MTS)–Lys(Z)–Leu–$N_3$

4,8 g de Boc–Arg(MTS)–Lys(Z)–Leu–NH–$NH_2$ (5,6 × $10^{-3}$ mole) sont dissous dans 100 ml de DMF. La solution obtenue est portée entre −15 et −20 °C sous agitation. On ajoute alors 2,9 ml d'une solution 7,75 N d'HCl dans le dioxanne, et tout de suite après 0,85 ml de nitrite de t-butyle. Le milieu est agité 40 min entre −15 et −20 °C sous atmosphère d'azote. On neutralise à pH 6,5–6,9 par la DIPEA. On prélève 15 ml de cette solution (0,25 équivalent) que l'on conserve à −20 °C sous azote. Le reste est engagé dans l'étape suivante.

b) Couplage de l'azide

9,83 g du peptide obtenu dans l'exemple XI, 8° sont dissous dans 40 ml de DMSO anhydre. On ajoute ensuite 60 ml de DMF. La solution est refroidie à −10 °C et le pH amené à 6,6 par addition de DIPEA. Cette solution est ensuite coulée en 15 min de la précédente solution dazide du tripeptide tout en maintenant la température entre −15 et −20 °C. Après fin de l'addition, le pH est amené à 7 par addition de DIPEA et conservé 24 h entre −15 et −20 °C. Après cet intervalle de temps, on rajoute le reste de la solution d'azide sur (15 ml) et le pH est ramené à 7 par addition de DIPEA et on conserve 24 h de plus entre −15 et −20 °C. Le milieu est ensuite versé sur 500 ml d'acétate d'éthyle refroidi et bien agité. Un solide blanc précipite lentement. Il est essorté et lavé 3 fois avec 150 ml d'acétate d'éthyle, puis à l'éther (150 ml × 2) et séché sous vide jusqu'à poids constant. On obtient ainsi 9,65 g (80,4%) d'une poudre blanche.

CCM: $BPEW_1$ – Rf: 0,54

AAA: Leu: 2,96 – Glu(Gln): 5,86 – Asp(Asn): 1,96 – Ile: 0,90 – Met: 0,91 – Ser: 1,86 – Arg: 4,86 – Gly: 2,06 – Lys: 0,93 – Ala: 2,00 –

10. H–Arg(MTS)–Lys(Z)–Leu–Leu–Gln–Asp(OBz1)–Ile–Met–Ser–Arg–Gln–Gln–Gly–Glu(OBz1)–Ser–Asn–Gln–Glu(OBz1)–Arg–Gly–Ala–Arg–Ala–Arg–Leu–$NH_2$, TFA, HCl

8 g du produit précédent (Exemple XI, 9°) sont mis en suspension dans 50 ml de chlorure de méthylène froid. On ajoute sous agitation 7 ml d'éthane dithiol et, en dernier, 70 ml de TFA. Le milieu est conservé 40 min à T.A., concentré sous vide de moitié et versé sur 500 ml d'éther. Le précipité obtenu est essoré, lavé 3 fois avec 200 ml d'éther et séché sous vide jusqu'à poids constant. Il est utilisé tel quel pour le couplage suivant.

Exemple XII

Synthèse de: Boc–Ile–Phe–Asn–Thr–Ser–Tyr–Arg–Lys(Z)–Val–Leu–Gly–Gln–Leu–Ser–Ala–OH, HCl (peptide J)

1. H–Gln–Leu–Ser–Ala–$OCH_3$, TFA

10,3 g (18,8 mM) de Boc–Gln–Leu–Ser–Ala–$OCH_3$ sont agités 35 min dans 100 ml de dichlorométhane contenant 100 ml de TFA. Le milieu réactionnel est concentré sous vide à environ 50 ml et versé sur 300 ml d'éther. Le précipité obtenu est essoré et lavé avec 3 fois 100 ml d'éther. Il est ensuite séché sous vide jusqu'à poids constant. Le rendement est quantitatif (10,4 g) et le produit est utilisé tel quel dans le couplage suivant.

2. Boc–Tyr–Arg(Z)–Val–Leu–Gly–Gln–Leu–Ser–Ala–$OCH_3$, HCl

Au sel de TFA obtenu ci-dessus, dissous dans 200 ml de diméthylformamide et neutralisé par de la N-éthylmorpholine, on ajoute 18,9 g (18,8 mM) de Boc–Tyr–Arg–Lys(Z)–Val–Leu–Gly–OH, HCl puis 9,2 g de BOP (21 mM) puis de la NEM pour amener le pH du milieu réactionnel vers 6. Après 4 h d'agitation, le milieu réactionnel est versé sur un litre d'AcOEt. Le précipité obtenu est filtré, lavé 3 fois par 100 ml d'AcOEt, puis 3 fois par 100 ml d'$Et_2O$ puis il est séché sous vide. On obtient 24,15 g de produit.

Rendement: 90%

CCM Rf: 0,20 ($BEW_1$) – alpha D: −17,6° – (Cc: 1 – DMF à 5% d'AcOH) – Produit identifié par 1H RMN et AAA: Ser: 1,01 – Glu: 0,99 – Gly: 1,06 – Ala: 1,01 – Val: 0,98 – Leu: 1,97 – Tyr: 1,00 – Lys: 0,98 – Arg: 0,99 – HPLC en phase reverse (EPP: 92%) –

3. H–Tyr–Arg–Lys(Z)–Val–Leu–Gly–Gln–Leu–Ser–Arg–$OCH_3$, HCl, TFA

8,66 g (6,1 mM) du produit ci-dessus sont agités 35 min dans 37 ml de dichlorométhane plus 3,5 ml d'anisole plus 40 ml de TFA. Après concentration sous vide de moitié, le résidu est versé sur 300 ml d'éther glacé; le précipité blanc obtenu est filtré, lavé à l'éther puis séché sous vide sur potasse.

Poids: 8,68 g

Rendement: 100%.

4. Boc–Ile–Phe–Thr–Asn–Ser–$N_3$

5,94 g (8,55 mM) de Boc–Ile–Phe–Thr–Asn–Ser–NH–$NH_2$ sont dissous dans 27 ml de DMSO plus 37 ml de DMF. Après refroidissement à −25 °C, on ajoute 6,1 ml d'une solution HCl/dioxanne 5,6 N puis 1,23 ml de nitrite de tertiobutyle. (1,2 équivalent) prédilué dans 5 ml de DMF prérefroidi à −20 °C. Après 1 h 15 min d'agitation entre −20 et −25 °C on ajoute 6,1 ml de diisopropyléthylamine (DIPEA) pour amener le pH vers 6, puis on ajoute 3 ml de DMF pour amener le volume à 85 ml et on conserve cette solution à −25 °C.

5. Boc–Ile–Phe–Thr–Asn–Ser–Tyr–Arg–Lys(Z)–Val–Leu–Gly–Gln–Leu–Ser–Ala–$OCH_3$, HCl

Le sel de TFA obtenu à l'exemple XI, 3. est dissous dans 70 ml de DMF et neutralisé par de la DIPEA puis ajouté en 15 min à 70 ml de la solution d'azide obtenue ci-dessus à −20 °C. Puis on ajoute de la DIPEA pour amener le pH vers 7, on agite une heure à −20 °C, puis on conserve le milieu à −12 °C. Après 22 h, on ajoute 5 ml de la solution d'azide et remonte le pH vers 7 par DIPEA. Après 45 h, on rajoute le restant de la solution d'azide et remonte le pH vers 7 par DIPEA. Après 74 h, le milieu réactionnel est versé sur 1200 ml d'acétate d'éthyle. Le précipité blanc obtenu est filtré, lavé à l'acétate d'éthyle puis à l'éther, puis séché sous vide.

Poids: 10,46 g – Rendement: 86% –

Par concentration des eaux-mères et reprécipitation par un mélange acétate d'éthyle-éther, on obtient un deuxième jet qui permet d'avoir un rendement global de 95%. Le produit de couplage est identifié par $^1H$ RMN et AAA: Asp: 1,02 – Thr: 1,03 – Ser: 1,91 – Glu: 0,97 – Gly: 1,06 – Ala: 1,06 – Val: 1,00 – Ile: 0,94 – Leu: 1,98 – Tyr: 1,04 – Phe: 1,02 – Lys: 0,94 – Arg: 1,02 – CCM Rf: 0,20 ($BEW_1$) – HPLC en phase reverse (EPP: 90%) –

6. Boc–Ile–Phe–Thr–Asn–Ser–Tyr–Arg–Lys(Z)–Val–Leu–Gly–Gln–Leu–Ser–Ala–OH, HCl

5,3 g (2,7 mM) du produit obtenu ci-dessus sont dissous dans 40 ml de DMSO plus 6 ml d'eau; on ajoute goutte à goutte 10 ml de soude IN, on agite 30 min puis neutralise par 10 ml d'acide chlorhydrique 1N. Le milieu réactionnel est versé sur un litre d'acétate d'éthyle. Le précipité obtenu est filtré, lavé 4 fois à l'acétate d'éthyle puis 2 fois à l'eau, puis 4 fois à l'éther, puis il est séché sous vide sur $P_2O_5$.

Poids: 4,56 g

Rendement: 86%

Produit identifié par $^1$N RMN et AAA – CCM: Rf: 0,5 $BEW_1$ –

Exemple XIII

Synthèse de Z–Tyr–Ala–Asp(OBz)–Ala–Ile–Phe–Thr–Asn–Ser–Tyr–Arg–Lys(Z)–Val–Leu–Gly–Gln–Leu–Ser–Ala–Arg(MTS)–Lys(Z)–Leu–Leu–Gln–Asp(OBz1)–Ile–Met–Ser–Arg–Gln–Gln–Gln–Gly–Glu(OBz1)–Ser–Asn–Gln–Glu(Obz1)–Arg–Gly–Ala–Arg–Ala–Arg–Leu–$NH_2$, HCl (GRF 1–44 protégé)

1. Boc–Ile–Phe–Thr–Asn–Ser–Tyr–Arg–Lys(Z)–Val–Leu–Gly–Gln–Leu–Ser–Ala–Arg(MTS)–Lys(Z)–Leu–Leu–Gln–Asp(OBz1)–Ile–Met–Ser–Arg–Gln–Gln–Gly–Glu(OBz1)–Ser–Asn–Gln–Glu(OBzl)–Arg–Gly–Ala–Arg–Ala–Arg–Leu–$NH_2$, HCl

6,6 g du sel de TFA du penta ecosa peptide obtenu dans l'exemple XI, 10. (1,9 × $10^{-3}$ mole) sont dissous dans 36 ml de DMSO. On ajoute 90 ml de DMF et le pH du milieu est ramené à 7 par addition de NEM. On ajoute ensuite 1,0 g de BOP, puis 3,8 g (1,9 × $10^{-3}$ mole) du peptide de l'exemple XII, 6°. Le pH du milieu est ramené à 6 par addition de NEM. Après 20 h d'agitation à la T.A. et sous atmosphère inerte ($N_2$), on rajoute 0,5 g de BOP. 12 h plus tard la réaction est terminée et le milieu réactionnel versé sur 600 ml d'acétate d'éthyle. Le prcipité obtenu est filtré et lavé avec l'acétate d'éthyle (3 fois 100 ml) et séché sous vide jusqu'à poids constant.

Le rendement est de 9,5 g (90%).

Le produit est identifié par HPLC et AAA.

2. H–Ile–Phe–Thr–Asn–Ser–Tyr–Arg–Lys(Z)–Val–Leu–Gly–Gln–Leu–Ser–Ala–Arg(MTS)–Lys(Z)–Leu–Leu–Gln–Asp(OBz1)–Ile–Met–Ser–Arg–Gln–Gln–Gly–Glu(OBz1)–Ser–Asn–Gln–Glu(OBz1)–Arg–Gly–Ala–Arg–Ala–Arg–Leu–$NH_2$, HCl, TFA

9,1 g du produit ci-dessus sont agités 35 min dans 100 ml de TFA plus 5 ml d'éthane dithiol. Puis le milieu réactionnel est versé sur 500 ml d'éther glacé. Le précipité obtenu est filtré, lavé par $Et_2O$, puis séché sous vide sur potasse.

Rendement: 100%

3. GRF–1–44 protégé

A 9,10 g du précédent sel de TFA (1,66 mM) en solution dans 50 ml de DMF et 50 ml de DMSO et neutralisé par la NEM, on ajoute 1,18 g du peptide décrit dans l'exemple X, (1,78 mM) et 0,79 g de BOP (1,78 mM); le pH est maintenu vers 6 par de la NEM. Après 23 h à TA, le milieu réactionnel est versé sur 600 ml d'AcOEt. Le précipité formé est filtré, lavé à l'acétate d'éthyle, puis à l'éther et finalement séché sous vide.

Rendement: 8,54 g (84%)

Contrôles RMN et CCM : AAA : Asp : 4,2 – Thr : 0,92 – Ser : 4,02 – Glu : 7,37 (pour 7) – Gly : 3,14 (pour 3) – Ala : 5,11 – Val : 1,01 – Met : 0,61 – Ile : 1,84 – Leu : 4,98 – Tyr – 1,87 – Phe : 0,95 – Arg : 5,90 –

Exemple XIV

Déprotection du GRF 1–44:

4,5 g du produit obtenu selon l'exemple XIII, 4°, sont agités une heure à 0 °C dans 180 ml de TFA contenant 19,8 ml de TFMSA, 27 ml de thionisole et 11 ml de métacrésol. On ajoute ensuite 1 l d'éther. Le précipité obtenu est filtré, lavé à l'éther puis séché une heure sous vide en présence de potasse. Le produit est redissous dans 200 ml d'eau et on ajoute de la résine échangeuse d'ions Amberlite IR 45 (sous forme acétate) afin de ramener le pH à 4,5. On agite le milieu 30 min à TA. La résine est filtrée, lavée à l'eau et les filtrats concentrés à 50 ml puis lyophilisés. Rendement : 3,85 g. Contrôle par RMN de la disparition des groupements protecteurs Z et OBzl.

CCM ($BPEW_1$) : tache majoritaire Rf 0,38

Pureté polypeptidique déterminée par HPLC : 65%.

Exemple XV

Purification du GRF 1–44

Le peptide déprotégé obtenu dans l'exemple précédent (XIV) est soumis à une chromatographie sur gel de Séphadex G 50 (fine) en utilisant l'acide acétique à 30% comme éluant. Les fractions contenant le peptide attendu sont réunies, évaporées et lyophilisées. Le lyophilisat ainsi obtenu est purifié à son tour par une chromatographie sur un échangeur de cations du type CM-32 carboxyméthylcellulose (Whatman) en utilisant un gradient linéaire d'acétate d'ammonium compris entre 0,1 M (pH 4,5) et 0,4 M (pH 6,5). Par exemple pour 1 charge de 1 g de peptide à purifier, on utilisera une colonne à chromatographie ayant une volume de lit de 50 ml environ pour une hauteur de 20 cm. Les fractions contenant le peptide avec un degré de pureté ⩾ à 80% (HPLC) sont réunies et lyophilisées jusqu'à poids constant (afin d'éliminer le tampon $CH_3CO_2NH_4$). Enfin, le précédent lyophilisat est soumis à une chromatographie de partition en utilisant comme support de la phase liquide stationnaire, le Séphadex G 50 fine et à l'aide du système de solvants suivant : n-butanol/éthanol/pyridine/acide acétique 0,2 N dans la proportion de 4/1/1/7 (en volumes). Les fractions de chromatographie sont contrôlées par HPLC et celles dont le titre de pureté est ⩾ à 95% sont réunies et lyophilisées.

Le rendement en produit isolé est de 35%.

Le produit est finalement contrôlé par analyse des amino acides:

| | | |
|---|---|---|
| Tyr : 1,91 (2) | Ser : 3,88 (4) | |
| Ala : 4,88 (5) | Arg : 5,89 (6) | |
| Asn, Asp : 3,86 (4) | Lys : 1,98 (2) | Gln, Glu : 6,82 (7) |
| Ile : 1,96 (2) | Val : 1,01 (1) | Met : 0,91 (1) |
| Phe : 0,93 (1) | Leu : 5,10 (5) | |
| Thr : 1,02 (1) | Gly : 2,98 (3) | |

**Revendications pour les Etats contractants: BE, CH, DE, FR, GB, IT, LI, LU, NL, SE**

1. Procédé de synthèse, en phase liquide et par fragments, des hGRF 1–44 et hGRF 1–40, caractérisé en ce qu'il consiste à coupler, l'un après l'autre et dans l'ordre de la séquence du GRF,

1) d'une part, les fragments ci-après:

| | | | |
|---|---|---|---|
| H–Ala–Arg–Ala–Arg–Leu–$NH_2$ | dit Fragment A | hGRF | (40–44) ou l'alaninamide (40) |
| H–Gln–Glu–Arg–Gly–OH | dit Fragment $B'_1$ | hGRF | (36–39) |
| H–Glu–Ser–Asn–OH | dit Fragment $B'_2$ | hGRF | (33–35) |
| H–Ser–Arg–Gln–Gln–Gly–OH | dit Fragment C | hGRF | (28–32) |
| H–Leu–Gln–Asp–Ile–Met–OH | dit Fragment D' | hGRF | (23–27) |
| H–Arg–Lys–Leu–OH | dit Fragment $E'_1$ | hGRF | (20–22) |

pour obtenir le peptide $K_1$ [(kGRF (20–44) ] ou le peptide correspondant ayant la séquence (20–40) et

2) d'autre part, les fragments ci-après:

| | | | |
|---|---|---|---|
| H–Gln–Leu–Ser–Ala | dit Fragment $F_1$ | hGRF | (16–19) |
| H–Tyr–Arg–Lys–Val–Leu–Gly–OH | dit Fragment $G_1$ | hGRF | (10–15) |
| H–Ile–Phe–Thr–Asn–Ser–OH | dit Fragment $H_1$ | hGRF | ( 5– 9) |

pour obtenir le peptide J [hGRF (5–19) ] et à coupler ensuite ensemble les peptides J et $K_1$ pour former le peptide ayant la séquence hGRF (5–44) ou hGRF (5–40) et finalement à coupler le peptide ainsi obtenu avec le peptide H–Tyr–Ala–Asp–Ala–OH dit fragment I hGRF (1–4), ledit procédé étant caractérisé en outre en ce que, dans lesdits fragments ci-dessus:

a) les fonctions acides latérales des acides aspartique et glutamique et la fonction amine latérale de la lysine sont protégées par des groupes protecteurs stables dans les conditions de déprotection du groupement Boc (tertiobutyloxycarbonyle);

b) la fonction guanidine de l'arginine en position 20 est protégée par le groupement MTS (triméthyl-2,4,6 phénylsulfonyle) et la fonction guanidine des autres arginines est protégée par protonation; et

c) l'acide aminé N-terminal est protégé sur l'amine par le groupement Boc, le groupement Boc de l'amine N-terminale du peptide en phase d'élongation étant éliminé sélectivement par hydrolyse à l'acide trifluoroacétique, ledit couplage étant effectué dans un solvant polaire aprotique et en fin de séquence tous les groupes protecteurs sont éliminés par hydrolyse à l'aide d'une solution 0,1 à 1 M d'acide méthanesulfonique ou trifluorométhanesulfonique dans l'acide trifluoroacétique.

2. Procédé selon la revendication 1, caractérisé en ce qu'après chaque couplage le produit obtenu est isolé du milieu réactionnel par précipitation à l'aide d'un tiers solvant insolubilisant.

3. Procédé selon la revendication 1, caractérisé en ce que le produit brut de réaction est purifié par la distribution à contre-courant et la chromatographie de perméation sur gel.

4. Procédé selon l'une quelconque des revendications 1 à 3, caractérisé en ce que le couplage est réalisé à l'aide de l'hexafluorophosphate de benzotriazolyloxyphosphonium (BOP) ou la dicyclohexyl-carbodiimide en présence d'hydroxy-1 benzotriazole (agent de couplage) ou par activation aux carboxyazides, dans un solvant approprié, tel quele diméthylformamide ou le diméthylsulfoxyde.

5. A titre de produits nouveaux, les peptides intermédiaires ou fragments peptidiques mis en œuvre dans le procédé selon l'une quelconque des revendications 1 à 4.

6. Peptides intermédiaires selon la revendication 5, caractérisés en ce qu'ils sont l'un des peptides ci-après:

- Boc – Gln – Glu(O Bzl) – Arg – Gly – OH
- X – Gln – Glu – Arg – Gly – OH
- Boc – Glu – (O Bzl) – Ser – Asn – OH
- X – Glu – Ser – Asn – OH
- Boc – Arg (MTS) – Lys (Z) – Leu – O $CH_3$
- X – Arg (MTS) – Lys (Z) – Leu – NH – $NH_2$
- X – Gln – Glu (O Bzl) – Arg – Gly – Ala – Arg – Ala – Arg – Leu – $NH_2$
- X – Glu (O Bzl) – Ser – Asn – Gln – Glu (O Bzl) – Arg – Gly – Ala – Arg – Ala – Arg – Leu – $NH_2$
- X – Ser – Arg – Gln – Gln – Gly – Glu (O Bzl) – Ser – Asn – Gln – Glu (O Bzl) – Arg – Gly – Ala – Arg – Ala – Arg – Leu – $NH_2$

•X – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$

•X – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$

•X – Ile – Phe – Thr – Asn – Ser – Tyr – Arg – Lys (Z) – Val – Leu – Gly – Gln – Leu – Ser – Ala – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$, dans lesquelles X représente H ou Boc.

**Revendications pour l'Etat contractant AT**

1. Procédé de synthèse, en phase liquide et par fragments, des hGRF 1–44 et hGRF 1–40, caractérisé en ce qu'il consiste à coupler, l'un après l'autre et dans l'ordre de la séquence du GRF,

1) d'une part, les fragments ci-après:

| | | | |
|---|---|---|---|
| H–Ala–Arg–Ala–Arg–Leu–$NH_2$ | dit Fragment A | hGRF | (40–44) ou l'alaninamide (40) |
| H–Gln–Glu–Arg–Gly–OH | dit Fragment $B'_1$ | hGRF | (36–39) |
| H–Glu–Ser–Asn–OH | dit Fragment $B'_2$ | hGRF | (33–35) |
| H–Ser–Arg–Gln–Gln–Gly–OH | dit Fragment C | hGRF | (28–32) |
| H–Leu–Gln–Asp–Ile–Met–OH | dit Fragment D' | hGRF | (23–27) |
| H–Arg–Lys–Leu–OH | dit Fragment $E'_1$ | hGRF | (20–22) |

pour obtenir le peptide $K_1$ [ (hGRF (20–44) ] ou le peptide correspondant ayant la séquence (20–40) et

2) d'autre part, les fragments ci-après:

| | | | |
|---|---|---|---|
| H–Gln–Leu–Ser–Ala | dit Fragment $F_1$ | hGRF | (16–19) |
| H–Tyr–Arg–Lys–Val–Leu–Gly–OH | dit Fragment $G_1$ | hGRF | (10–15) |
| H–Ile–Phe–Thr–Asn–Ser–OH | dit Fragment $H_1$ | hGRF | ( 5– 9) |

pour obtenir le peptide J [hGRF (5–19) ] et à coupler ensuite ensemble les peptides J et $K_1$ pour former le peptide ayant la séquence hGRF (5–44) ou hGRF (5–40) et finalement à coupler le peptide ainsi obtenu avec le peptide H–Tyr–Ala–Asp–Ala–OH dit fragment I hGRF (1–4), ledit procédé étant caractérisé en outre en ce que, dans lesdits fragments ci-dessus:

a) les fonctions acides latérales des acides aspartique et glutamique et la fonction amine latérale de la lysine sont protégées par des groupes protecteurs stables dans les conditions de déprotection du groupement Boc (tertiobutyloxycarbonyle);

b) la fonction guanidine de l'arginine en position 20 est protégée pa le groupement MTS (triméthyl-2,4,6 phénylsulfonyle) et la fonction guanidine des autres arginines est protégée par protonation; et

c) l'acide aminé N-terminal est protégé sur l'amine par le groupement Boc, le groupement Boc de l'amine N-terminale du peptide en phase d'élongation étant éliminé sélectivement par hydrolyse à l'acide trifluoroacétique, ledit couplage étant effectué dans un solvant polaire aprotique et en fin de séquence tous les groupes protecteurs sont éliminés par hydrolyse à l'aide d'une solution 0,1 à 1 M d'acide méthanesulfonique ou trifluorométhanesulfonique dans l'acide trifluoroacétique.

2. Procédé selon la revendication 1, caractérisé en ce qu'après chaque couplage le produit obtenu est isolé du milieu réactionnel par précipitation à l'aide d'un tiers solvant insolubilisant.

3. Procédé selon la revendication 1, caractérisé en ce que le produit brut de réaction est purifié par la distribution à contre-courant et la chromatographie de perméation sur gel.

4. Procédé selon l'une quelconque des revendications 1 à 3, caractérisé en ce que le couplage est réalisé à l'aide de l'hexafluorophosphate de benzotriazolyloxyphosphonium (BOP) ou la dicyclohexyl-carbodiimide en présence d'hydroxy-1 benzotriazole (agent de couplage) ou par activation aux carboxyazides, dans un solvant approprié, tel que le diméthylformamide ou le diméthylsulfoxyde.

**Patentansprüche für die Vertragsstaaten: BE, CH, DE, FR, GB, IT, LI, LU, NL, SE**

1. Verfahren zur Synthese von hGRF 1–44 und hGRF 1–40 in flüssiger Phase und mittels Fragmenten, dadurch gekennzeichnet, dass es in der Kopplung nacheinander und in der Reihenfolge der Sequenz des GRF

1) einerseits der nachstehenden Fragmente:

| | | | |
|---|---|---|---|
| H–Ala–Arg–Ala–Arg–Leu–$NH_2$, | genannt Fragment A | hGRF | (40–44) oder Alaninamid (40) |
| H–Gln–Glu–Arg–Gly–OH, | genannt Fragment $B'_1$ | hGRF | (36–39) |
| H–Glu–Ser–Asn–OH, | genannt Fragment $B'_2$ | hGRF | (33–35) |
| H–Ser–Arg–Gln–Gln–Gly–OH, | genannt Fragment C | hGRF | (28–32) |
| H–Leu–Gln–Asp–Ile–Met–OH, | genannt Fragment D' | hGRF | (23–27) |
| H–Arg–Lys–Leu–OH, | genannt Fragment $E'_1$ | hGRF | (20–22) |

zur Darstellung des Peptids $K_1$ [hGRF (20–44) ] oder des entsprechenden Peptids mit der Sequenz (20–40), und

2) anderseits der nachstehenden Fragmente:

| | | | |
|---|---|---|---|
| H–Gln–Leu–Ser–Ala, | genannt Fragment $F_1$ | hGRF | (16–19) |
| H–Tyr–Arg–Lys–Val–Leu–Gly–OH, | genannt Fragment $G_1$ | hGRF | (10–15) |
| H–Ile–Phe–Thr–Asn–Ser–OH, | genannt Fragment $H_1$ | hGRF | ( 5– 9) |

zur Darstellung des Peptids J [hGRF (5–19) ] und in der anschliessenden Kopplung der Gesamtheit der Peptide J und $K_1$ zur Bildung des Peptids mit der Sequenz hGRF (5–44) oder hGRF (5–40) und schliesslich in der Kopplung des so erhaltenen Peptids mit dem Peptid H–Tyr–Ala–Asp–Ala–OH, genannt Fragment I hGRF (1–4), besteht, welches Verfahren weiters dadurch gekennzeichnet ist, dass in den oben angeführten Fragmenten:

a) die seitlichen Säurefunktionen der Asparagin- und Glutaminsäure und die seitliche Aminfunktion von Lysin durch unter Entschützungsbedingungen der Gruppe Boc (tert. Butyloxycarbonyl) stabile Schutzgruppen geschützt sind;

b) die Guanidinfunktion von Arginin in Position 20 durch die Gruppe MTS (2,4,6-Trimethylphenylsulfonyl) geschützt ist und die Guanidinfunktion der anderen Arginine durch Protonierung geschützt ist;

c) die N-terminale Aminosäure am Amin durch die Gruppe Boc geschützt ist, welche Gruppe Boc des N-terminalen Amins des Peptids in der Verlängerungsphase durch Hydrolyse mit Trifluoressigsäure selektiv entfernt wird, wobei die Kopplung in einem aprotischen polaren Lösungsmittel durchgeführt wird und am Ende der Sequenz sämtliche Schutzgruppen durch Hydrolyse mit einer 0,1 bis 1M Lösung Methansulfonsäure oder Trifluormethansulfonsäure in Trifluoressigsäure entfernt werden.

2. Verfahren nach Anspruch 1, dadurch gekennzeichnet, dass nach jeder Kopplung das erhaltene Produkt durch Ausfällung mit Hilfe eines dritten, nicht lösenden Lösungsmittels vom Reaktionsmilieu isoliert wird.

3. Verfahren nach Anspruch 1, dadurch gekennzeichnet, dass das Rohprodukt der Reaktion durch Verteilung im Gegenstrom und durch Gel-Permeationschromatographie gereinigt wird.

4. Verfahren nach einem der Ansprüche 1 bis 3, dadurch gekennzeichnet, dass die Kopplung mit Hilfe von Benzotriazolyloxyphosphonium-hexafluorphosphat (BOP) oder Dicyclohexylcarbodiimid in Gegenwart von 1-Hydroxybenzotriazol (Kopplungsmittel) oder durch Aktivierung mit Carboxyaziden in einem geeigneten Lösungsmittel, wie Dimethylformamid oder Dimethylsulfoxid, durchgeführt wird.

5. Als neue Produkte die intermediären Peptide oder Peptidfragmente, die beim Verfahren nach einem der Ansprüche 1 bis 4 eingesetzt werden.

6. INtermediäre Peptide nach Anspruch 5, dadurch gekennzeichnet, dass sie eines der nachstehenden Peptide sind:

- Boc – Gln – Glu(O Bzl) – Arg – Gly – OH
- X – Gln – Glu – Arg – Gly – OH
- Boc – Glu – (O Bzl) – Ser – Asn – OH
- X – Glu – Ser – Asn – OH
- Boc – Arg (MTS) – Lys (Z) – Leu – O $CH_3$
- X – Arg (MTS) – Lys (Z) – Leu – NH – $NH_2$
- X – Gln – Glu (O Bzl) – Arg – Gly – Ala – Arg – Ala – Arg – Leu – $NH_2$
- X – Glu (O Bzl) – Ser – Asn – Gln – Glu (O Bzl) – Arg – Gly – Ala – Arg – Ala – Arg – Leu – $NH_2$
- X – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Ile – Phe – Thr – Asn – Ser – Tyr – Arg – Lys (Z) – Val – Leu – Gly – Gln – Leu – Ser – Ala – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$, worin X für H oder Boc steht.

## Patentansprüche für den Vertragsstaat AT

1. Verfahren zur Synthese von hGRF 1–44 und hGRF 1–40 in flüssiger Phase und mittels Fragmenten, dadurch gekennzeichnet, dass es in der Kopplung nacheinander und in der Reihenfolge der Sequenz des GRF

1) einerseits der nachstehenden Fragmente:

| | | | |
|---|---|---|---|
| H–Ala–Arg–Ala–Arg–Leu–$NH_2$, | genannt Fragment A | hGRF | (40–44) oder Alaninamid (40) |
| H–Gln–Glu–Arg–Gly–OH, | genannt Fragment $B'_1$ | hGRF | (36–39) |
| H–Glu–Ser–Asn–OH, | genannt Fragment $B'_2$ | hGRF | (33–35) |
| H–Ser–Arg–Gln–Gln–Gly–OH, | genannt Fragment C | hGRF | (28–32) |
| H–Leu–Gln–Asp–Ile–Met–OH, | genannt Fragment D' | hGRF | (23–27) |
| H–Arg–Lys–Leu–OH, | genannt Fragment $E'_1$ | hGRF | (20–22) |

zur Darstellung des Peptids $K_1$ [hGRF (20–44) ] oder des entsprechenden Peptids mit der Sequenz (20–40), und

2) anderseits der nachstehenden Fragmente:

| | | | |
|---|---|---|---|
| H–Gln–Leu–Ser–Ala, | genannt Fragment $F_1$ | hGRF | (16–19) |
| H–Tyr–Arg–Lys–Val–Leu–Gly–OH, | genannt Fragment $G_1$ | hGRF | (10–15) |
| H–Ile–Phe–Thr–Asn–Ser–OH, | genannt Fragment $H_1$ | hGRF | ( 5– 9) |

zur Darstellung des Peptids J [hGRF (5–19) ] und in der anschliessenden Kopplung der Gesamtheit der Peptide J und $K_1$ zur Bildung des Peptids mit der Sequenz hGRF (5–44) oder hGRF (5–40) und schliesslich in der Kopplung des so erhaltenen Peptids mit dem Peptid H–Tyr–Ala–Asp–Ala–OH, genannt Fragment I hGRF (1–4), besteht, welches Verfahren weiters dadurch gekennzeichnet ist, dass in den oben angeführten Fragmenten:

a) die seitlichen Säurefunktionen der Asparagin- und Glutaminsäure und die seitliche Aminfunktion von Lysin durch unter Entschützungsbedingungen der Gruppe Boc (tert. Butyloxycarbonyl) stabile Schutzgruppen geschützt sind;

b) die Guanidinfunktion von Arginin in Position 20 durch die Gruppe MTS (2,4,6-Trimethylphenylsulfonyl) geschützt ist und die Guanidinfunktion der anderen Arginine durch Protonierung geschützt ist;

c) die N-terminale Aminosäure am Amin durch die Gruppe Boc geschützt ist, welche Gruppe Boc des N-terminalen Amins des Peptids in der Verlängerungsphase durch Hydrolyse mit Trifluoressigsäure selektiv entfernt wird, wobei die Kopplung in einem aprotischen polaren Lösungsmittel durchgeführt wird und am Ende der Sequenz sämtliche Schutzgruppen durch Hydrolyse mit einer 0,1 bis 1M Lösung Methansulfonsäure oder Trifluormethansulfonsäure in Trifluoressigsäure entfernt werden.

2. Verfahren nach Anspruch 1, dadurch gekennzeichnet, dass nach jeder Kopplung das erhaltene Produkt durch Ausfällung mit Hilfe eines dritten, nicht lösenden Lösungsmittels vom Reaktionsmilieu isoliert wird.

3. Verfahren nach Anspruch 1, dadurch gekennzeichnet, dass das Rohprodukt der Reaktion durch Verteilung im Gegenstrom und durch Gel-Permeationschromatographie gereinigt wird.

4. Verfahren nach einem der Ansprüche 1 bis 3, dadurch gekennzeichnet, dass die Kopplung mit Hilfe von Benzotriazolyloxyphosphonium-hexafluorphosphat (BOP) oder Dicyclohexylcarbodiimid in Gegenwart von 1-Hydroxybenzotriazol (Kopplungsmittel) oder durch Aktivierung mit Carboxyaziden in einem geeigneten Lösungsmittel, wie Dimethylformamid oder Dimethylsulfoxid, durchgeführt wird.

**Claims for the contracting States: BE, CH, DE, FR, GB, IT, LI, LU, NL, SE**

1. Process for the synthesis, in liquid phase and by fragments, of hGRF 1–44 and hGRF 1–40, characterized in that it consists in coupling, one after the other and in the order of the sequence of the GRF,

1) on the one hand, the following fragments:

| | | | |
|---|---|---|---|
| H–Ala–Arg–Ala–Arg–Leu–$NH_2$ | called Fragment A | hGRF | (40–44) or alaninamide (40) |
| H–Gln–Glu–Arg–Gly–OH | called Fragment $B'_1$ | hGRF | (36–39) |
| H–Glu–Ser–Asn–OH | called Fragment $B'_2$ | hGRF | (33–35) |
| H–Ser–Arg–Gln–Gln–Gly–OH | called Fragment C | hGRF | (28–32) |
| H–Leu–Gln–Asp–Ile–Met–OH | called Fragment D' | hGRF | (23–27) |
| H–Arg–Lys–Leu–OH | called Fragment $E'_1$ | (hGRF | (20–22) |

to obtain the peptide $K_1$ [hGRF (20–44) ] or the corresponding peptide having the sequence (20–40) and

2) on the other hand, the following fragments:

| | | | |
|---|---|---|---|
| H–Gln–Leu–Ser–Ala | called Fragment $F_1$ | hGRF | (16–19) |
| H–Tyr–Arg–Lys–Val–Leu–Gly–OH | called Fragment $G_1$ | hGRF | (10–15) |
| H–Ile–Phe–Thr–Asn–Ser–OH | called Fragment $H_1$ | hGRF | ( 5– 9) |

to obtain the peptide J [hGRF (5–19) ] and thereafter to couple together the peptides J and $K_1$ in order to form the peptide having the sequence hGRF (5–44) or hGRF (5–40) and finally to couple the thus obtained peptide with the peptide H–Tyr–Ala–Asp–Ala–OH, called fragment I hGRF (1–4), said process being further characterized in that, in said above fragments:

a) the side acid functions of the aspartic and glutamic acids and the side amine function of the lysine are protected by protector groups stable in the conditions of deprotection of the group Boc-(tertiobutyloxycarbonyl);

b) the guanidine function of the arginine in position 20 is protected by the MTS group (2,4,6-trimethyl pehnylsulfonyl) and the guanidine function of the other arginines is protected by protonation; and

c) the N-terminal amino acid is protected on the amine by the Boc group, the group Boc from the N-terminal amine of the peptide in phase of elongation being selectively eliminated by hydrolysis with trifluoroacetic acid, said coupling being effected in an aprotic solvent and, at the end of sequence, all the protector groups are eliminated by hydrolysis with the aid of a 0,1 to 1M solution of methanesulfonic or trifluoromethanesulfonic acid in trifluoroacetic acid.

2. Process according to claim 1, characterized in that after each coupling, the product obtained is isolated from the reaction medium by precipitation with the aid of a third insolubilizing solvent.

3. Process according to claim 1, characterized in that the crude reaction product is purified by counter-current distribution and permeation chromatography over gel.

4. Process according to any one of claims 1 to 3, characterized in that the coupling is achieved with the help of hexafluorophosphate of benzotriazolyloxyphosphonium (BOP) or dicyclohexylcarbodiimide in the presence of 1-hydroxy-benzotriazole (coupling agent) or by activation with carboxyazides, in an appropriate solvent such as dimethylformamide or dimethylsulfoxyde.

5. As novel products, the intermediate peptides or peptide fragments used in the process claimed in any one of claims 1 to 4.

6. Intermediate peptides according to claim 5, characterized in that said peptides are one of the following:

- Boc – Gln – Glu(O Bzl) – Arg – Gly – OH
- X – Gln – Glu – Arg – Gly – OH
- Boc – Glu – (O Bzl) – Ser – Asn – OH
- X – Glu – Ser – Asn – OH
- Boc – Arg (MTS) – Lys (Z) – Leu – O $CH_3$
- X – Arg (MTS) – Lys (Z) – Leu – NH – $NH_2$
- X – Gln – Glu (O Bzl) – Arg – Gly – Ala – Arg – Ala – Arg – Leu – $NH_2$
- X – Glu (O Bzl) – Ser – Asn – Gln – Glu (O Bzl) – Arg – Gly – Ala – Arg – Ala – Arg – Leu – $NH_2$
- X – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$
- X – Ile – Phe – Thr – Asn – Ser – Tyr – Arg – Lys (Z) – Val – Leu – Gly – Gln – Leu – Ser – Ala – Arg (MTS) – Lys (Z) – Leu – Leu – Gln – Asp (O Bzl) – Ile – Met – Ser – Arg – Gln – Gln – Gly – Glu (O Bzl) – Ser – Asn – Gln – Glu (O Bzl) – Arg – Gly – Ala – Arg – Ala – Arg – Leu – $NH_2$, in which X represents H or Boc.

**Claims for the contracting State AT**

1. Process for the synthesis, in liquid phase and by fragments, of hGRF 1–44 and hGRF 1–40, characterized in that it consists in coupling, one after the other and in the order of the sequence of the GRF,

1) on the one hand, the following fragments:

| | | | |
|---|---|---|---|
| H–Ala–Arg–Ala–Arg–Leu–$NH_2$ | called Fragment A | hGRF | (40–44) or alaninamide (40) |
| H–Gln–Glu–Arg–Gly–OH | called Fragment $B'_1$ | hGRF | (36–39) |
| H–Glu–Ser–Asn–OH | called Fragment $B'_2$ | hGRF | (33–35) |
| H–Ser–Arg–Gln–Gln–Gly–OH | called Fragment C | hGRF | (28–32) |
| H–Leu–Gln–Asp–Ile–Met–OH | called Fragment D' | hGRF | (23–27) |
| H–Arg–Lys–Leu–OH | called Fragment $E'_1$ | hGRF | (20–22) |

to obtain the peptide $K_1$ [hGRF (20–44) ] or the corresponding peptide having the sequence (20–40) and

2) on the other hand, the following fragments:

| | | | |
|---|---|---|---|
| H–Gln–Leu–Ser–Ala | called Fragment $F_1$ | hGRF | (16–19) |
| H–Tyr–Arg–Lys–Val–Leu–Gly OH | called Fragment $G_1$ | hGRF | (10–15) |
| H–Ile–Phe–Thr–Asn–Ser–OH | called Fragment $H_1$ | hGRF | ( 5– 9) |

to obtain the peptide J [hGRF (5–19) ] and thereafter to couple together the peptides J and $K_1$ in order to form the peptide having the sequence hGRF (5–44) or hGRF (5–40) and finally to couple the thus obtained peptide with the peptide H–Tyr–Ala–Asp–Ala–OH, called fragment I hGRF (1–4), said process being further characterized in that, in said above fragments:

a) the side acid functions of the aspartic and glutamic acids and the side amine function of the lysine are protected by protector groups stable in the conditions of deprotection of the group Boc (tertiobutyloxycarbonyl);

b) the guanidine function of the arginine in position 20 is protected by the MTS group (2,4,6-trimethyl phenylsulfonyl) and the guanidine function of the other arginines is protected by protonation; and

c) the N-terminal amino acid is protected on the amine by the Boc group, the group Boc from the N-terminal amine of the peptide in phase of elongation being selectively eliminated by hydrolysis with trifluoroacetic acid, said coupling being effected in an aprotic solvent and, at the end of sequence, all the protector groups are eliminated by hydrolysis with the aid of a 0,1 to 1M solution of methanesulfonic or trifluoromethanesulfonic acid in trifluoroacetic acid.

2. Process according to claim 1, characterized in that after each coupling, the product obtained is isolated from the reaction medium by precipitation with the aid of a third insolubilizing solvent.

3. Process according to claim 1, characterized in that the crude reaction product is purified by

counter-current distribution and permeation chromatography over gel.

4. Process according to any one of claims 1 to 3, characterized in that the coupling is achieved with the help of hexafluorophosphate of benzotriazolyloxyphosphonium (BOP) or dicyclohexylcarbodiimide in the presence of 1-hydroxy-benzotriazole (coupling agent) or by activation with carboxyazides, in an appropriate solvent such as dimethylformamide or dimethylsulfoxyde.